(19)

Europäisches
Patentamt
European
Patent Office
Office européen
des brevets

(11)  **EP 4 318 478 A1**

(12) **EUROPEAN PATENT APPLICATION**
published in accordance with Art. 153(4) EPC

(43) Date of publication:
**07.02.2024  Bulletin 2024/06**

(21) Application number: **22778504.5**

(22) Date of filing: **09.03.2022**

(51) International Patent Classification (IPC):
*G16B 40/00* (2019.01)

(52) Cooperative Patent Classification (CPC):
**G16B 15/30; G06N 5/01; G06N 20/20; G16B 20/50; G16B 40/20**

(86) International application number:
**PCT/CN2022/079885**

(87) International publication number:
**WO 2022/206320 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **01.04.2021  CN 202110355929**

(71) Applicant: **Tencent Technology (Shenzhen) Company Limited**
**Shenzhen, Guangdong, 518057 (CN)**

(72) Inventors:
• **YANG, Ziyi**
  **Shenzhen, Guangdong 518057 (CN)**
• **YE, Zhaofeng**
  **Shenzhen, Guangdong 518057 (CN)**
• **LIAO, Benben**
  **Shenzhen, Guangdong 518057 (CN)**
• **ZHANG, Sheng Yu**
  **Shenzhen, Guangdong 518057 (CN)**

(74) Representative: **EP&C**
**P.O. Box 3241**
**2280 GE Rijswijk (NL)**

(54)  **PREDICTION MODEL TRAINING AND DATA PREDICTION METHODS AND APPARATUSES, AND STORAGE MEDIUM**

(57)    This application relates to a method and apparatus for training a prediction model, a computer device and a storage medium. The method includes: acquiring a training sample set, the training sample set including a plurality of training samples, a training sample weight corresponding to each of the training samples, and target energy characteristics corresponding to each of the training samples; determining a current training sample from the training sample set based on the training sample weight; inputting current target energy characteristics corresponding to the current training sample into a pre-trained prediction model for basic training, and obtaining a basic prediction model after completing the basic training; and updating the training sample weight corresponding to each of the training samples based on the basic prediction model in an iteration way, and in a case of reaching a model training completion condition, the basic prediction model reaching the model training completion condition being treated as a target prediction model, the target prediction model being configured to predict interaction state information corresponding to inputted protein information and inputted compound information. With the adoption of the method, the prediction accuracy of the target prediction model obtained by training can be improved.

FIG. 2

**Description**

RELATED APPLICATION

This application claims priority to Chinese Patent Application No. 2021103559296, entitled "METHODS AND APPARATUSES FOR TRAINING PREDICTION MODEL AND PREDICTING DATA AND STORAGE MEDIUM" filed with National Intellectual Property Administration on April 01, 2021, which is incorporated herein by reference in its entirety.

FIELD OF THE TECHNOLOGY

This application relates to the field of computer technology, and particularly relates to methods and apparatuses for training a prediction model and predicting data, a computer device and a storage medium.

BACKGROUND OF THE DISCLOSURE

With the development of artificial intelligence technology, the affinity between a compound and a targeted protein can be predicted by a machine learning algorithm. At present, a model established by the machine learning algorithm is used for predicting the change of affinity between the targeted protein and the compound after the mutation, and then determining whether the targeted protein has drug resistance to the compound, so as to provide a reference for doctors to use drugs. However, the prediction model established by the machine learning algorithm has the problems of low accuracy and poor generalization ability.

SUMMARY

In view of the above technical problems, this application provides methods and apparatuses for training a prediction model and predicting data, a computer device and a storage medium, aiming at improving the prediction model training accuracy so as to improve the prediction accuracy.
A method for training a prediction model includes:

acquiring a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information;

determining a current training sample from the training sample set based on the training sample weight;

inputting current target energy characteristics corresponding to the current training sample into a pre-trained prediction model for basic training, and obtaining a basic prediction model after completing the basic training; and

judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model, the target prediction model being configured to predict interaction state information corresponding to the inputted protein information and the inputted compound information.

An apparatus for training a prediction model includes:

a sample acquisition module, configured to acquire a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information;

a sample determination module, configured to determine a current training sample from the training sample set based on the training sample weight;

a training module, configured to input the current target energy characteristics corresponding to the current training sample into a pre-trained prediction model for basic training, and obtaining a basic prediction model after completing the basic training; and

an iteration module, configured to judge whether a model training completion condition is reached or not; in a case of not reaching the model training completion condition, update the training sample weight corresponding to each of the training samples based on the basic prediction model, and return to perform the operation of determining the current training sample from the training sample set based on the training sample weight; in a case of reaching the model training completion condition, treat the basic prediction model reaching the model training completion condition as a target prediction model, the target prediction model being configured to predict interaction state information corresponding to the inputted protein information and the inputted compound information.

A computer device is provided, including a memory and a processor, the memory storing computer-readable instructions, the processor, when executing the computer-readable instructions, implementing the following steps:

acquiring a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information;

determining a current training sample from the training sample set based on the training sample weight;

inputting current target energy characteristics corresponding to the current training sample into a pre-trained prediction model for basic training, and obtaining a basic prediction model after completing the basic training; and

judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model, the target prediction model being configured to predict interaction state information corresponding to the inputted protein information and the inputted compound information.

A computer-readable storage medium is provided, storing computer-readable instructions, the computer-readable instructions, when executed by a processor, implementing the following steps:

acquiring a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information;

determining a current training sample from the training sample set based on the training sample weight;

inputting current target energy characteristics corresponding to the current training sample into a pre-trained prediction model for basic training, and obtaining a basic prediction model after completing the basic training; and

judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model, the target prediction model being configured to predict interaction state information corresponding to the inputted protein information and the inputted compound information.

According to the method and apparatus for training a prediction module, the computer device and the storage medium, the training sample set is acquired, the training sample set includes a plurality of training samples, the training sample weight corresponding to each of the training samples and the target energy characteristics corresponding to each of the training samples, each of the training samples includes the wild type protein information, the mutant type protein information and the compound information, and the current training sample is determined from the training sample set based on the training sample weight; the current target energy characteristics corresponding to the current training sample are inputted into the pre-trained prediction model for basic training, and the basic prediction model is obtained after completing the basic training; and it is judged whether a model training completion condition is reached or not; in a case of not reaching the model training completion condition, the training sample weight corresponding to each of the training samples is updated based on the basic prediction model, and the operation of determining the current training sample from the training sample set is performed based on the training sample weight; in a case of reaching the model training completion condition, the basic prediction model reaching the model training completion condition is treated as a target prediction model, the target prediction model is configured to predict the interaction state information corresponding to the inputted protein information and the inputted compound information. That is, the training sample weight is continuously updated in the iteration process, the current training sample is determined from the training sample set by using the training sample weight, thus the quality of the training sample can be ensured; and then the prediction model is trained by using the current training sample, so that the prediction accuracy and generalization of the target prediction model obtained by training can be improved.

A method for predicting data includes:

acquiring to-be-predicted data, the to-be-predicted data including to-be-predicted wild type protein information, to-be-predicted mutant type protein information and to-be-predicted compound information;

performing binding energy characteristic extraction based on the to-be-predicted wild type protein information and the to-be-predicted compound information to obtain to-be-predicted wild type energy characteristics, and performing binding energy characteristic extraction based on the to-be-predicted mutant type protein information and the to-be-predicted compound information to obtain to-be-predicted mutant type energy characteristics;

determining to-be-predicted target energy characteristics based on the to-be-predicted wild type energy characteristics and the to-be-predicted mutant type energy characteristics;

inputting the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model.

An apparatus for predicting data includes:

a data acquisition module, configured to acquire to-be-predicted data, the to-be-predicted data including to-be-predicted wild type protein information, to-be-predicted mutant type protein information and to-be-predicted compound information;

a characteristic extraction module, configured to perform binding energy characteristic extraction based on the to-be-predicted wild type protein information and the to-be-predicted compound information to obtain to-be-predicted wild type energy characteristics, and perform binding energy characteristic extraction based on the to-be-predicted mutant type protein information and the to-be-predicted compound information to obtain to-be-predicted mutant type energy characteristics;

a target characteristic determination module, configured to determine to-be-predicted target energy characteristics based on the to-be-predicted wild type energy characteristics and the to-be-predicted mutant type energy characteristics; and

a prediction module, configured to input the to-be-predicted target energy characteristics into a target prediction model for prediction to obtain interaction state information; the target prediction model being obtained by following operations: acquiring a training sample set; determining a current training sample from a training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model.

A computer device is provided, including a memory and a processor, the memory storing computer-readable instructions, the processor, when executing the computer-readable instructions, implementing the following steps:

acquiring to-be-predicted data, the to-be-predicted data including to-be-predicted wild type protein information, to-be-predicted mutant type protein information and to-be-predicted compound information;

performing binding energy characteristic extraction based on the to-be-predicted wild type protein information and the to-be-predicted compound information to obtain to-be-predicted wild type energy characteristics, and performing binding energy characteristic extraction based on the to-be-predicted mutant type protein information and the to-be-predicted compound information to obtain to-be-predicted mutant type energy characteristics;

determining to-be-predicted target energy characteristics based on the to-be-predicted wild type energy characteristics and the to-be-predicted mutant type energy characteristics;

inputting the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model.

A computer-readable storage medium is provided, storing computer-readable instructions, the computer-readable instructions, when executed by a processor, implementing the following steps:

acquiring to-be-predicted data, the to-be-predicted data including to-be-predicted wild type protein information, to-be-predicted mutant type protein information and to-be-predicted compound information;

performing binding energy characteristic extraction based on the to-be-predicted wild type protein information and the to-be-predicted compound information to obtain to-be-predicted wild type energy characteristics, and performing binding energy characteristic extraction based on the to-be-predicted mutant type protein information and the to-be-predicted compound information to obtain to-be-predicted mutant type energy characteristics;

determining to-be-predicted target energy characteristics based on the to-be-predicted wild type energy characteristics and the to-be-predicted mutant type energy characteristics;

inputting the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model.

According to the method and apparatus for predicting data, the computer device and the storage medium, the to-be-predicted data is acquired, the to-be-predicted target energy characteristics are determined, and the to-be-predicted target energy characteristics are inputted into the target prediction model for prediction to obtain the interaction state information; the target prediction model is obtained by following operations: acquiring a training sample set; determining a current training sample from the training sample set based on the training sample weight; inputting the current target energy characteristics corresponding to the current training sample into the pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model, that is, the interaction state information is obtained by predicting through the target prediction model; and the target prediction model obtained by training can improve the prediction accuracy so that the accuracy of the obtained interaction state information can be improved.

BRIEF DESCRIPTION OF THE DRAWINGS

To describe the technical solutions in embodiments of this application more clearly, the following briefly describes the accompanying drawings required for describing the embodiments. Apparently, the accompanying drawings in the following description show merely some embodiments of this application, and a person of ordinary skill in the art may derive other drawings from these accompanying drawings without creative efforts.

FIG. 1 is an application environment diagram of a method for training a prediction model in one embodiment.

FIG. 2 is a schematic flow diagram of a method for training a prediction model in one embodiment.

FIG. 3 is a schematic flow diagram of a pre-trained initial prediction model in one embodiment.

FIG. 4 is a schematic flow diagram of obtaining initial interaction state information in one embodiment.

FIG. 5 is a schematic flow diagram of obtaining target energy characteristics in one embodiment.

FIG. 6 is a schematic flow diagram of obtaining wild type energy characteristics in one embodiment.

FIG. 7 is a schematic flow diagram of obtaining mutant type energy characteristics in one embodiment.

FIG. 8 is a schematic flow diagram of obtaining a target basic prediction model in one embodiment.

FIG. 9 is a schematic flow diagram of obtaining a basic prediction model in one embodiment.

FIG. 10 is a schematic flow diagram of obtaining an updated sample weight in one embodiment.

FIG. 11 is a schematic flow diagram of a method for predicting data in one embodiment.

FIG. 12 is a schematic flow diagram of an application scene of a method for predicting data in a specific embodiment.

FIG. 13 is a schematic flow diagram of a method for training a prediction model in a specific embodiment.

FIG. 14 is a schematic flow diagram of a method for training a prediction model in a specific embodiment.

FIG. 15 is a schematic diagram of a comparison test result in a specific embodiment.

FIG. 16 is a schematic diagram of accuracy and recall rate curve indexes in a specific embodiment in FIG. 15.

FIG. 17 is a structural block diagram of an apparatus for training a prediction model in one embodiment.

FIG. 18 is a structural block diagram of an apparatus for predicting data in one embodiment.

FIG. 19 is a diagram of an internal structure of a computer device according to an embodiment.

FIG. 20 is a diagram of an internal structure of a computer device according to another embodiment.

DESCRIPTION OF EMBODIMENTS

To make the objectives, technical solutions, and advantages of this application clearer and more understandable, this application is further described in detail below with reference to accompanying drawings and embodiments. It is to be understood that the specific embodiments described herein are only used for explaining this application, and are not used for limiting this application.

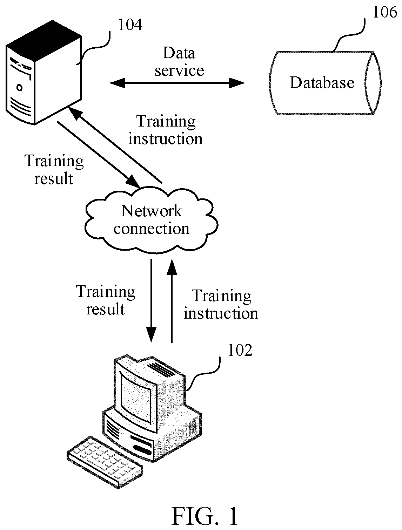

A method for training a prediction model provided in this application may be applied to an application environment shown in FIG. 1. A terminal 102 communicates with a server 104 through a network. The server 104 receives a model training instruction transmitted from the terminal 102. The server 104 acquires a training sample set from a database 106 according to the model training instruction. The training sample set includes a plurality of training samples, a training sample weight corresponding to each of the training samples and target energy characteristics corresponding to each of the training samples. Each of the training samples includes wild type protein information, mutant type protein information and compound information. The target energy characteristics are obtained based on wild type energy characteristics and mutant type energy characteristics. The wild type energy characteristics are obtained by performing binding energy characteristic extraction based on the wild type protein information and the compound information. The mutant type energy characteristics are obtained by performing binding energy characteristic extraction based on the mutant type protein information and the compound information. The server 104 determines a current training sample from the training sample set based on the training sample weight. The server 104 inputs the current target energy characteristics corresponding to the current training sample into a pre-trained prediction model for basic training, and obtains a basic prediction model after completing the basic training. The server 104 updates the training sample weight corresponding to each of the training samples based on the basic prediction model, and returns to perform the operation of determining the current training sample from the training sample set based on the training sample weight until completing model training to obtain a target prediction model. The target prediction model is configured to predict interaction state information corresponding to the inputted protein information and the inputted compound information. The terminal 102 may be, but not limited to, a personal computer, a notebook computer, a smartphone, a tablet computer, and a portable wearable device. The server 104 may be implemented by an independent server, or a server cluster that includes a plurality of servers.

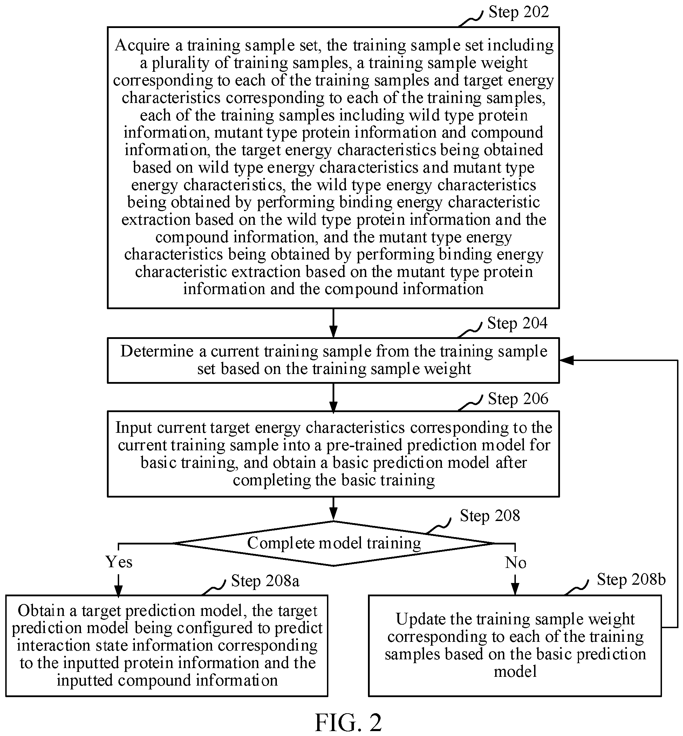

In the embodiments of the present disclosure, including the embodiments of the claims and the specification (hereinafter referred to as all embodiments of the present disclosure), as shown in FIG. 2, an exemplary method for training a prediction model is provided. The application of this method is described by taking the server in FIG. 1 as an example. It is to be understood that this method may also be applied to the terminal and may also be applied to a system including the terminal and the server, and the method is implemented through interaction of the terminal and the server. In this embodiment, the method includes the following steps:

Step 202: Acquire a training sample set, the training sample set including a plurality of training samples, a training sample weight corresponding to each of the training samples and the target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information.

The protein refers to targeted protein, such as protein kinase. The compound refers to a drug capable of interacting with the targeted protein, such as a tyrosine kinase inhibitor. The protein information is used for characterizing specific information of the targeted protein, and may include a protein structure, protein physicochemical properties, etc. The wild type protein information refers to information of an individual obtained from the nature, namely non-artificially mutated protein. The mutant type protein information refers to mutated protein information, such as mutation of a drug structure. The compound information refers to information of a compound capable of interacting with the protein, and may include a structure of the compound, physicochemical properties of the compound, etc. The training sample weight refers to a weight corresponding to the training sample and is used for characterizing the quality of the corresponding training sample. The high-quality training sample can improve the training quality when training a machine learning model. The binding energy characteristics refer to characteristics obtained when the protein and the compound interact with each other and are used for characterizing interaction energy information between targeted protein and compound molecules, and may include structural characteristics, physicochemical property characteristics, energy characteristics, etc. The binding energy characteristics are characteristics obtained after characteristic selection. The wild type energy characteristics refer to binding energy characteristics obtained when the wild type protein and the compound interact with each other. The mutant type energy characteristics refer to binding energy characteristics obtained when the mutant type protein and the compound interact with each other. The target energy characteristics are used for characterizing a difference between the mutant type energy characteristics and the wild type energy characteristics.

Specifically, the server may directly acquire the training sample set from the database. The training sample set includes a plurality of training samples, the training sample weight corresponding to each of the training samples and the target energy characteristics corresponding to each of the training samples. Each of the training samples includes the wild type protein information, the mutant type protein information and the compound information. The target energy characteristics are obtained based on the wild type energy characteristics and the mutant type energy characteristics. The wild type energy characteristics are obtained by performing binding energy characteristic extraction based on the wild type protein information and the compound information. The mutant type energy characteristics are obtained by performing binding energy characteristic extraction based on the mutant type protein information and the compound information. The server may further collect each of the training samples from the Internet, extract the target energy characteristics corresponding to each of the training samples, and initialize the training sample weight corresponding to each of the training samples. The server may further acquire the training sample set from a third-party server which provides data service, for example, may acquire the training sample set from a third-party cloud server.

In all embodiments of the present disclosure, the server may acquire protein information, mutant type protein information and compound information, perform binding energy characteristic extraction based on the wild type protein information and the compound information to obtain the wild type energy characteristics, perform binding energy characteristic extraction based on the mutant type protein information and the compound information to obtain the mutant type energy characteristics, and compute the difference between the wild type energy characteristics and the mutant type energy characteristics to obtain the target energy characteristics. Meanwhile, initializing corresponding training sample weights may be, for example, random initialization, zero initialization, Gaussian distribution initialization, etc.

Step 204: Determine a current training sample from the training sample set based on the training sample weight.

The current training sample refers to a training sample used in current training.

Specifically, the server selects a training sample from the training sample set according to the training sample weight corresponding to each of the training samples to obtain the current training sample. For example, the training sample with the training sample weight larger than a preset weight threshold may be treated as the current training sample. The preset weight threshold is a weight threshold set in advance. In a specific embodiment, the training sample weight may be set to be 0 and 1, namely, the training sample weight corresponding to each of the training samples is initialized to be 0 or 1. In a case that the training sample weight is 1, the corresponding training sample is treated as the current training sample. In one embodiment, the server may select multiple training samples from the training sample set according to the training sample weight to obtain a current training sample set. The current training sample set includes multiple training samples. The current training sample set is used for training the basic prediction model.

Step 206: Input current target energy characteristics corresponding to the current training sample into a pre-trained prediction model for basic training, and obtain a basic prediction model after completing the basic training.

The current target energy characteristics refer to target energy characteristics corresponding to the current training sample. The pre-trained prediction model is a prediction model which is preliminarily trained in advance. The prediction model is established by using a random forest algorithm. The prediction model may be configured to predict the change of affinity between compounds and proteins before and after mutation. The basic prediction model is obtained by training through the corresponding current training samples under the condition that the training sample weight is kept unchanged.

Specifically, the server may input the current target energy characteristics corresponding to the previous training sample into the pre-trained prediction model for prediction to obtain a prediction result; compute loss according to the prediction result; reversely update the pre-trained prediction model according to the loss, and return to perform the operation of inputting the current target energy characteristics corresponding to the previous training sample into the pre-trained prediction model for prediction in an iteration way until reaching basic training completion conditions; and treating the prediction model reaching the basic training completion condition as the basic prediction model. The basic training completion condition refers to a condition for obtaining the basic prediction model, including that the training reaches a preset iteration frequency upper limit or the loss reaches a preset threshold, or parameters of the model are not changed any more, etc.

Step 208: Judge whether model training is completed or not (in some embodiments, judging whether a preset model training completion condition is reached or not), perform step 208a in a case that model training is completed (in some embodiments, in a case of reaching the model training completion condition), otherwise (in some embodiments, in a case of not reaching the model training completion condition), perform step 208b, and return to perform step 204.

Step 208a: Obtain a target prediction model (in some embodiments, the basic prediction model reaching the model training completion condition is treated as a target prediction model), the target prediction model being configured to predict interaction state information corresponding to the inputted protein information and the inputted compound information.

Step 208b: Update the training sample weight corresponding to each of the training samples based on the basic prediction model, and return to perform the operation of determining the current training sample from the training sample set based on the training sample weight.

The completion of the model training refers to reaching of the condition for obtaining the target prediction model. The target prediction model refers to a model which is obtained by final training and configured to predict the interaction state information corresponding to the inputted protein information and the inputted compound information. The interaction state information is used for characterizing the change of binding free energy between the compounds and the proteins before and after mutation. The binding free energy refers to the interaction between a ligand and a receptor.

Specifically, in a case of obtaining the basic prediction model, the server further judges whether the model training is completed or not. The model training completion condition may include that the iteration frequency reaches a preset model training iteration frequency upper limit. In a case of not reaching the model training completion condition, the parameters of the basic prediction model are kept unchanged at the moment. Then the training sample weight corresponding to each of the training samples is updated by using the basic prediction model. The target energy characteristics corresponding to each of the training samples may be inputted into the basic prediction model to obtain the loss corresponding to each of the training samples. The training sample weight corresponding to each of the training samples is updated according to the loss corresponding to each of the training samples. In a case of updating the training sample weight, the operation of determining the current training sample from the training sample set based on the training sample weight is performed again in a continuous iteration way until reaching the model training completion condition. The basic prediction model reaching the model training completion condition is treated as the target prediction model. The target prediction model configured to predict the interaction state information corresponding to the inputted protein information and the inputted compound information.

The prediction module training method includes: acquiring the training sample set, the training sample set including a plurality of training samples, the training sample weight corresponding to each of the training samples and target energy characteristics corresponding to each of the training samples, each of the training samples including the wild type protein information, the mutant type protein information and the compound information; determining the current training sample from the training sample set based on the training sample weight; inputting the current target energy characteristics corresponding to the current training sample into the pre-trained prediction model for basic training, and obtaining the basic prediction model after completing the basic training; and updating the training sample weight corresponding to each of the training samples based on the basic prediction model, and returning to perform the operation of determining the current training sample from the training sample set based on the training sample weight until completing model training to obtain the target prediction model, the target prediction model being configured to predict the interaction state information corresponding to the inputted protein information and the inputted compound information. That is, the training sample weight is continuously updated in the iteration process, the current training sample is determined from the training sample set by using the training sample weight, thus the quality of the training sample can be ensured; and then the prediction model is trained by using the current training sample, so that the prediction accuracy and generalization of the target prediction model obtained by training can be improved.

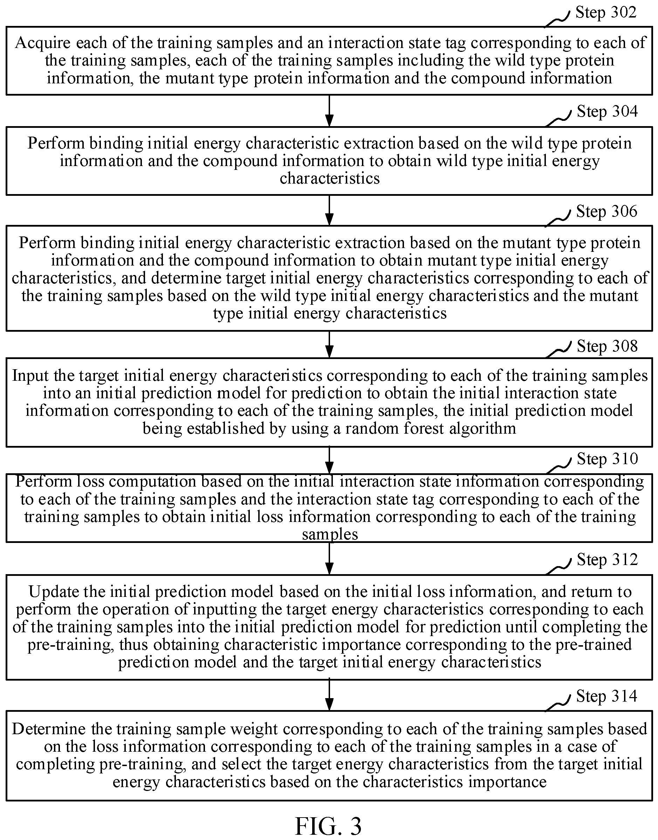

In all embodiments of the present disclosure, as shown in FIG. 3, before step 202 of acquiring a training sample set, the method can further include the following steps:

Step 302: Acquire each of the training samples, each of the training samples including the wild type protein information, the mutant type protein information and the compound information.

Step 304: Perform binding initial energy characteristic extraction based on the wild type protein information and the compound information to obtain wild type initial energy characteristics.

The binding initial energy characteristics refer to extracted unscreened characteristics, and may include non-physical model characteristics, physical and experience potential energy-based characteristics, etc. The non-physical model characteristics include crystal protein-compound structure characteristics, physicochemical property characteristics of ligands and residues, energy characteristics obtained by computing by an experience or descriptor-based scoring function, etc. The physical and experience potential energy-based characteristics refer to energy characteristics obtained by computing through a modeling program based on mixed physical and experience potential energy. The wild type initial energy characteristics refer to binding initial energy characteristics obtained when the wild type protein information and the compound information interact with each other.

Specifically, the server may acquire each of the training samples from the database. Each of the training samples may be the sample used in pre-training. Each of the training samples may be the same as or different from the training sample in the training sample set. The server may further collect each of the training samples from the Internet. The server may further acquire each of the training samples from a server which provides data service. Each of the training samples includes the wild type protein information, the mutant type protein information and the compound information. Meanwhile, the server performs characteristic extraction on each of the training samples, namely, performs binding initial energy characteristic extraction based on the wild type protein information and the compound information to obtain the wild type initial energy characteristics corresponding to each of the training samples.

Step 306: Perform binding initial energy characteristic extraction based on the mutant type protein information and the compound information to obtain mutant type initial energy characteristics, and determine target initial energy characteristics corresponding to each of the training samples based on the wild type initial energy characteristics and the mutant type initial energy characteristics.

The mutant type initial energy characteristics refer to the binding initial energy characteristics obtained when the mutant type protein information and the compound information interact with each other. The target initial energy characteristics are used for characterizing the difference between the wild type initial energy characteristics and the mutant type initial energy characteristics.

Specifically, the server performs binding initial energy characteristic extraction on the mutant type protein information and the compound information to obtain mutant type initial energy characteristics, and computes the difference between the wild type initial energy characteristics and the mutant type initial energy characteristics. The difference is treated as target initial energy characteristics. For example, the difference between structural characteristics may be computed and treated as the target structural characteristics. The difference between physicochemical properties may also be computed and treated as the target structural characteristics.

Step 308: Input the target initial energy characteristics corresponding to each of the training samples into an initial prediction model for prediction to obtain initial interaction state information corresponding to each of the training samples, the initial prediction model being established by using a random forest algorithm.

The initial prediction model is a prediction model with initialized model parameters. The model parameters may be initialized at any time, may also be subjected to zero initialization, etc. The initial prediction model is established by using the random forest algorithm. The random forests refer to a classifier for training and predicting samples by using multiple trees. For example, an ExtraTree (extreme random tree) algorithm may be used for establishing the initial prediction model. The initial interaction state information refers to interaction state information obtained by predicting through the initial prediction model.

Specifically, the server establishes the initial prediction model with initialized model parameters through the random forest algorithm in advance, and then inputs the target initial energy characteristics corresponding to each of the training samples into the initial prediction model for prediction to obtain the outputted initial interaction state information corresponding to each of the training samples.

Step 310: Perform loss computation based on the initial interaction state information corresponding to each of the training samples and the interaction state tag corresponding to each of the training samples to obtain initial loss information corresponding to each of the training samples.

The interaction state tag refers to real interaction state information. Each of the training samples has a corresponding interaction state tag. The initial loss information is used for characterizing the error between the initial interaction state information and the interaction state tag.

Specifically, the server computes the loss between the initial interaction state information corresponding to each of the training samples and the interaction state tag by using a preset loss function to obtain the initial loss information corresponding to each of the training samples. The loss function may be a mean square error loss function, an average absolute value error loss function, etc.

Step 312: Update the initial prediction model based on the initial loss information, and return to perform the operation of inputting the target energy characteristics corresponding to each of the training samples into the initial prediction model for prediction until completing the pre-training, thus obtaining characteristic importance corresponding to the pre-trained prediction model and the target initial energy characteristics.

The completion of pre-training refers to reaching of the condition for obtaining the pre-trained prediction model, namely, the pre-training reaches the preset iteration frequency, or the loss of pre-training reaches the preset threshold, or the prediction model parameters for pre-training are not changed any more. The characteristic importance is used for characterizing the importance degree of the target initial energy characteristics. The higher the characteristic importance is, the more important the corresponding characteristics are, and the more the contribution to model training is.

Specifically, the server computes a gradient by using the initial loss information, and then reversely updates the initial prediction model with the gradient to obtain the updated prediction model; judges whether the pre-training is completed; treats the updated prediction model as the initial prediction model in a case of not completing the pre-training, and returns to perform the operation of inputting the target energy characteristics corresponding to each of the training samples into the initial prediction model for prediction in an iteration way until completing the pre-training; and treats the updated prediction model obtained by the last iteration as the pre-trained prediction model, and directly obtains the characteristic importance corresponding to the target initial energy characteristics after training the pre-trained prediction model which is established by using the random forest algorithm. Each characteristic in the target initial energy characteristics has the corresponding characteristic importance.

Step 316: Determine the training sample weight corresponding to each of the training samples based on the loss information corresponding to each of the training samples in a case of completing pre-training, and select the target energy characteristics from the target initial energy characteristics based on the characteristic importance.

Specifically, the server may determine the training sample weight corresponding to each of the training samples through the loss information corresponding to each of the training samples after completing the pre-training, for example, may compare the loss information corresponding to each of the training samples with the weight loss threshold, and treats the corresponding training sample as a sample with good quality in a case that the loss information is greater than the weight loss threshold. The corresponding training sample weight may be set to be 1. In a case that the loss information is not greater than the weight loss threshold, the corresponding training sample is treated as a sample with poor quality. The corresponding training sample weight may be set to be 0. Characteristic selection is carried out on the target initial energy characteristics through the characteristic importance so as to obtain the target energy characteristics. The target energy characteristics are characteristics to be extracted during further training of the pre-trained prediction model.

In the above embodiment, pre-training is performed through each of the training samples to obtain the pre-trained model, then the training sample weight corresponding to each of the training samples is determined based on the loss information corresponding to each of the training samples after completing pre-training, and characteristic selection is performed on the target initial energy characteristics based on the characteristic importance to obtain the target energy characteristics. Therefore, the training efficiency can be improved in further training, and the accuracy of the training can be ensured.

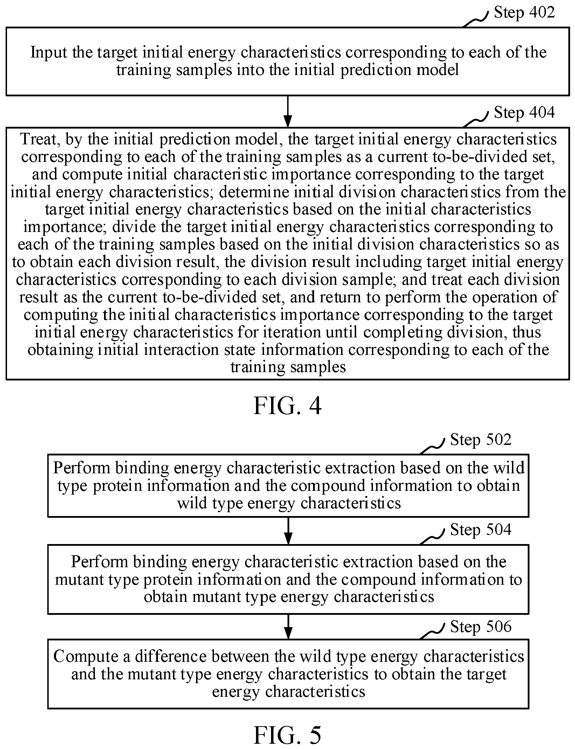

In all embodiments of the present disclosure, as shown in FIG. 4, step 308 of inputting the target initial energy characteristics corresponding to each of the training samples into an initial prediction model for prediction to obtain initial interaction state information corresponding to each of the training samples, the initial prediction model being established by using a random forest algorithm can include the following steps:

Step 402: Input the target initial energy characteristics corresponding to each of the training samples into the initial prediction model.

Step 404: Treat, by the initial prediction model, the target initial energy characteristics corresponding to each of the training samples as a current to-be-divided set and compute initial characteristic importance corresponding to the target initial energy characteristics; determine initial division characteristics from the target initial energy characteristics based on the initial characteristic importance; divide the target initial energy characteristics corresponding to each of the training samples based on the initial division characteristics so as to obtain each division result, the division result including target initial energy characteristics corresponding to each division sample; and treat each division result as the current to-be-divided set, and return to perform the operation of computing the initial characteristic importance corresponding to the target initial energy characteristics for iteration until completing division, thus obtaining initial interaction state information corresponding to each of the training samples.

The initial characteristic importance refers to characteristic importance corresponding to the target initial energy characteristics. The initial division characteristics refer to characteristics for decision tree division. The division result is obtained by dividing the target initial energy characteristics. The division sample refers to a training sample corresponding to the target initial energy characteristics in the division result.

Specifically, the server inputs the target initial energy characteristics corresponding to each of the training samples into the initial prediction model. The initial prediction model scores the input characteristics to obtain the initial characteristic importance corresponding to the target initial energy characteristics. The initial characteristic importance may be computed by using information gain, an information gain rate, a Gini coefficient, a mean square error, etc. The initial division characteristics are determined from the target initial energy characteristics based on the initial characteristic importance. The target initial energy characteristics corresponding to each of the training samples are divided based on the initial division characteristics. That is, the target initial energy characteristics exceeding the initial division characteristics are treated as one part, and the target initial energy characteristics not exceeding the initial division characteristics are treated as the other part, thus obtaining the division result. The division result includes the target initial energy characteristics corresponding to each division sample. Each division result is treated as the current to-be-divided set. The operation of computing the initial characteristic importance corresponding to the target initial energy characteristics is performed again for iteration until completing division, thus obtaining the initial interaction state information corresponding to each of the training samples. Division completion refers to that each tree node cannot be divided, that is, leaf nodes have the only corresponding target initial energy characteristic. The initial interaction state information refers to interaction state information predicted by the initial prediction model.

In the above embodiment, the target initial energy characteristics corresponding to each of the training samples are inputted into the initial prediction model. The initial prediction model computes the initial characteristic importance corresponding to the target initial energy characteristics; determines the initial division characteristics from the target initial energy characteristics based on the initial characteristic importance; divides the target initial energy characteristics corresponding to each of the training samples based on the initial division characteristics to obtain each division result, the division result including the target initial energy characteristics corresponding to each division sample; and treats each division result as the current to-be-divided set, and returns to perform the operation of computing the initial characteristic importance corresponding to the target initial energy characteristics for iteration until completing the division, thus obtaining the initial interaction state information corresponding to each of the training samples, and improving the accuracy of the obtained initial interaction state information.

In all embodiments of the present disclosure, step 202 of acquiring a training sample set, the training sample set including the training sample weight corresponding to each of the training samples can include:
acquiring confidence corresponding to each of the training samples, and determining the training sample weight corresponding to each of the training samples based on the confidence.

The confidence is used for characterizing the quality of the corresponding training sample. The higher the confidence is, the higher the quality corresponding to the training sample is, and the better the performance of a model obtained by training with the training sample having high confidence is.

Specifically, the server may also acquire the confidence corresponding to each of the training samples while acquiring each of the training samples. Then, the confidence may be directly treated as the training sample weight corresponding to each of the training samples. The confidence may be manually set or obtained by carrying out confidence evaluation on each of the training samples in advance. In one embodiment, the confidence corresponding to each of the training samples may also be compared with the preset confidence threshold. In a case of exceeding the confidence threshold, the corresponding training sample weight is set to be 1, and this training sample is treated as the current training sample. In a case of not exceeding the confidence threshold, the corresponding training sample weight is set to be 0.

In the above embodiment, the confidence is acquired, and the training sample weight corresponding to each of the training samples is determined according to the confidence, thus improving the efficiency of obtaining the training sample weight.

In all embodiments of the present disclosure, as shown in FIG. 5, step 202 of acquiring a training sample set, the training sample set including the target energy characteristics corresponding to each of the training samples can include the following steps:

Step 502: Perform binding energy characteristic extraction based on the wild type protein information and the compound information to obtain wild type energy characteristics.

Step 504: Perform binding energy characteristic extraction based on the mutant type protein information and the compound information to obtain mutant type energy characteristics.

The wild type energy characteristics include, but are not limited to, the wild type protein characteristics, the compound characteristics, and the energy characteristics obtained when the wild type protein information and the compound information interact with each other. The wild type protein characteristics are used for characterizing characteristics corresponding to the wild type protein information, including, but not limited to, the wild type protein structural characteristics and the wild type protein physicochemical property characteristics. The compound characteristics include, but are not limited to, the compound structural characteristics and the compound physicochemical property characteristics. The mutant type energy characteristics include, but are not limited to, the mutant type protein characteristics, the compound characteristics, and the energy characteristics obtained when the mutant type protein information and the compound information interact with each other. The mutant type protein characteristics are used for characterizing characteristics corresponding to the mutant type protein information, including, but not limited to, the mutant type protein structural characteristics and the mutant type protein physicochemical property characteristics.

Specifically, the server performs characteristic extraction through the wild type protein information and the compound information to extract the wild type protein characteristics and compound characteristics, extracts the energy characteristics obtained when the wild type protein and compounds interact with each other, and treats the wild type protein characteristics, the compound characteristics and the energy characteristics as the wild type energy characteristics. The server performs characteristic extraction through the mutant type protein information to obtain the mutant type protein characteristics, then extracts the energy characteristics obtained when the mutant type protein and the compounds interact with each other, and treats the extracted mutant type protein characteristics, the compound characteristics and the energy characteristics as the mutant type energy characteristics.

Step 506: Compute a difference between the wild type energy characteristics and the mutant type energy characteristics to obtain the target energy characteristics.

Specifically, the server computes the difference between the wild type energy characteristics and the mutant type energy characteristics, for example, the difference between the wild type protein characteristics and the mutant type protein characteristics, and computes the difference between the energy characteristics obtained when the wild type protein and the compounds interact with each other and the energy characteristics obtained when the mutant type protein and the compounds interact with each other so as to obtain the target energy characteristics. In a specific embodiment, a characteristic difference value between the wild type energy characteristics and the mutant type energy characteristics may be computed to obtain the target energy characteristics.

In the above embodiment, the wild type energy characteristics and the mutant type energy characteristics are extracted, and then the difference between the wild type energy characteristics and the mutant type energy characteristics is computed to obtain the target energy characteristics, thus improving the accuracy of the obtained target energy characteristics.

In all embodiments of the present disclosure, the wild type energy characteristics can include first wild type energy characteristics and second wild type energy characteristics;

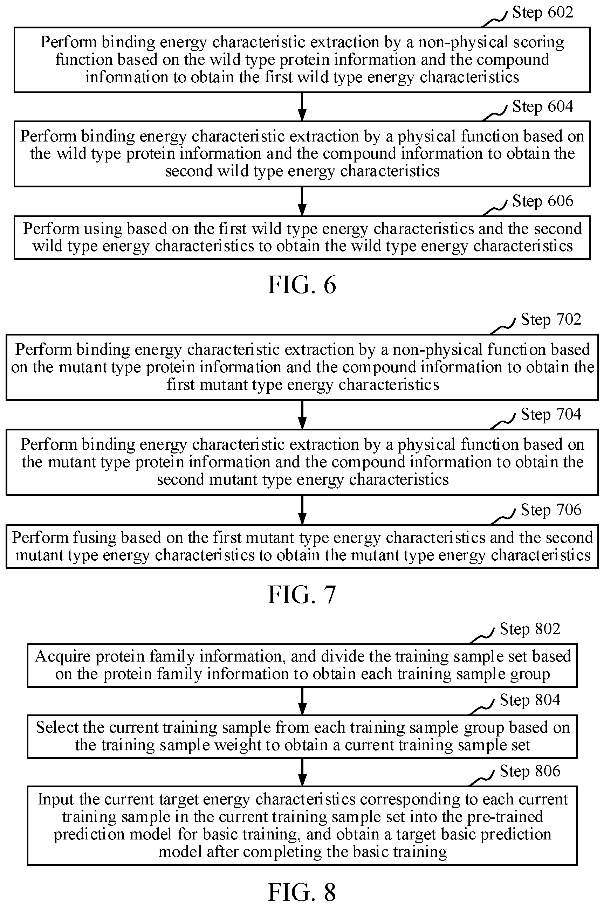

As shown in FIG. 6, step 502 of performing binding energy characteristic extraction based on the wild type protein information and the compound information to obtain wild type energy characteristics includes the following steps:

Step 602: Perform binding energy characteristic extraction by a non-physical scoring function based on the wild type protein information and the compound information to obtain the first wild type energy characteristics.

The non-physical scoring function refers to an experience or descriptor-based scoring function. The scoring function is on the basis of some prior assumptions or used for fitting experimental data to obtain the energy characteristics. The obtained energy characteristics do not have obvious interpretable physical significance. The first wild type energy characteristics refer to first part of the extracted energy characteristics.

Specifically, the server may perform binding energy characteristic extraction by using a preset non-physical scoring function, compute the wild type protein information and the compound information by using the non-physical scoring function to obtain a computing result, and treat the computing result as the first wild type energy characteristics. The scoring function (a function for evaluating the reasonability of a theoretically obtained receptor-ligand combination mode) may be used for extracting the energy characteristics.

Step 602: Perform binding energy characteristic extraction by a physical function based on the wild type protein information and the compound information to obtain the second wild type energy characteristics.

The physical function refers to an energy function based on mixed physical and experience potential energy, and the physical function has obvious physical significance. An energy function family is composed of a force field function based on experimental data fitting, a quantitative computing function based on a first principle, a solvent model based on a continuous medium, etc.

Specifically, the server performs binding energy characteristic extraction on the wild type protein information and the compound information through the preset physical function to obtain the second wild type energy characteristics. For example, the energy function in a modeling program Rosetta (a polymer modeling software library taking Monte Carlo simulated annealing as an algorithm core) based on the mixed physical and experience potential energy may be used for computing the energy characteristics.

Step 602: Perform fusing based on the first wild type energy characteristics and the second wild type energy characteristics to obtain the wild type energy characteristics.

Specifically, the server computes a characteristic difference value between the first wild type energy characteristics and the second wild type energy characteristics to obtain the wild type energy characteristics.

In the above embodiment, the first wild type energy characteristics and the second wild type energy characteristics are extracted, and fusing is performed based on the first wild type energy characteristics and the second wild type energy characteristics to obtain the wild type energy characteristics. The first wild type energy characteristics and the second wild type energy characteristics can better characterize the interaction energy information between the wild type targeted protein and the compound molecules so that the accuracy of the obtained wild type energy characteristics is improved.

In all embodiments of the present disclosure, the mutant type energy characteristics can include first mutant type energy characteristics and second mutant type energy characteristics.

As shown in FIG. 7, step 504 of performing binding energy characteristic extraction based on the mutant type protein information and the compound information to obtain mutant type energy characteristics includes the following steps:

Step 702: Perform binding energy characteristic extraction by a non-physical function based on the mutant type protein information and the compound information to obtain the first mutant type energy characteristics.

Step 704: Perform binding energy characteristic extraction by a physical function based on mutant type protein information and the compound information to obtain the second mutant type energy characteristics.

Specifically, the server performs binding energy characteristic extraction on the mutant type protein information and the compound information through the preset non-physical function to obtain the first mutant type energy characteristics, and then performs binding energy characteristic extraction on the mutant type protein information and the compound information through the preset physical function to obtain the second mutant type energy characteristics.

Step 706: Perform fusing based on the first mutant type energy characteristics and the second mutant type energy characteristics to obtain the mutant type energy characteristics.

Specifically, the server computes a characteristic difference value between the first mutant type energy characteristics and the second mutant type energy characteristics to obtain the mutant type energy characteristics.

In the above embodiment, the first mutant type energy characteristics and the second mutant type energy characteristics are extracted, and fusing is performed based on the first mutant type energy characteristics and the second mutant type energy characteristics to obtain the mutant type energy characteristics. The first mutant type energy characteristics and the second mutant type energy characteristics can better characterize the interaction energy information between the mutant targeted protein and the compound molecules so that the accuracy of the obtained mutant type energy characteristics is improved.

In all embodiments of the present disclosure, as shown in FIG. 8, step 204 of determining a current training sample from the training sample set based on the training sample weight can include the following steps:

Step 802: Acquire protein family information, and divide the training sample set based on the protein family information to obtain each training sample group.

Proteins with similar in-vivo amino acid sequences and very similar structures and functions form a "protein family", and members in the same protein family are called "homologous protein". The protein family information refers to the information of the protein family. The training sample group is obtained by gathering each of the training samples corresponding to the same protein family.

Specifically, the server directly acquires the protein family information from the database. The protein family information may also be obtained from the Internet or from a third-party server which provides the data service. In one embodiment, the server may further divide the protein family with similar structures or sequences of the protein information in the training sample into the same training sample group to obtain each training sample group.

Step 804: Select the current training sample from each training sample group based on the training sample weight to obtain a current training sample set.

Specifically, the server selects the current training sample from each training sample group by using the training sample weight, namely, sequentially selects the current training sample according to the training sample weight in the training sample group, and selects from each training sample group to obtain the current training sample set.

Step 206 of inputting current target energy characteristics corresponding to the current training sample into a pre-trained prediction model for basic training, and obtaining a basic prediction model after completing the basic training includes the following step:

Step 806: Input the current target energy characteristics corresponding to each current training sample in the current training sample set into the pre-trained prediction model for basic training, and obtain a target basic prediction model after completing the basic training.

Specifically, the server inputs the current target energy characteristics corresponding to each current training sample in the current training sample set into the pre-trained prediction model for basic training, and obtains the target basic prediction model after completing the basic training.

In the above embodiment, the training sample set is divided according to the protein family information to obtain each training sample group. Then, the current training sample is selected from each training sample group based on the training sample weight to obtain the current training sample set, and thus the basic training is performed on the pre-trained prediction model through the current training sample set to obtain the basic prediction model. That is, the current training sample is selected from each training sample group, and the selected training samples are distributed everywhere in the space rather than concentrated in a local area. Thus the global information contained in the training samples can be learned during model training, the comprehensiveness of the model in learning knowledge in the training process can be ensured, furthermore, the convergence speed in the model training process is improved, and the generalization ability of the model obtained by training is improved.

In a specific embodiment, the basic form of the pre-trained prediction model is shown in the following formula (1).

$$\min_{w \in \Gamma, v \in [0,1]^n} \sum_{i=1}^{n} v_i L\left(g_w(x_i), y_i\right) - \lambda \left\|v\right\|_1 - \gamma \left\|v\right\|_{2.1} \qquad \text{formula (1)}$$

$n$ represents a total number of training samples; $X$ represents a training sample set, $X = (x_1,..., x_n) \in R^{n*m}$, $R$ represents a real number set; and $m$ represents a number of energy characteristics. $x_i$ represents an $i^{th}$ training sample; and $y_i$ represents an interaction state tag corresponding to the $i^{th}$ training sample. $g$ represents a pre-trained prediction model; w represents model parameters; L represents a loss function; and v represents a training sample weight. $v = (v^{(1)},..., v^{(b)}$, b represents a number of the training sample groups, namely the training sample set is divided into b groups: $x^{(1)},..., x^{(b)}$, wherein $x^{(j)} \in R^{n_j*m}$ represents a training sample of a $j^{th}$ training sample group; in

$$v^{(j)} = \left(v_1^j,..., v_{n_j}^j\right) \in [0,1]^{n_j}$$

, $n_j$ represents a number of training samples in the $j^{th}$ training sample group, and in $\sum_{j=1}^{b} n_j = n$, $v_1^j$, $v_1^j$ represents a training sample weight corresponding to a $1^{st}$ training sample in the $j^{th}$ training sample group. $V_i$ represents an $i^{th}$ training sample weight. $\lambda$ represents parameters of training sample difficulty, namely, the training samples are sequentially selected from those which are easy to select (high confidence) to those which are difficult to select (low confidence). $\gamma$ represents parameters of sample diversity. That is, the samples are selected from multiple training sample groups. $\|\|_1$ represents $L_1$ norm, and $\|\|_{2.1}$ represents $L_{2.1}$ norm.

In $-\left\|v\right\|_{2.1} = -\sum_{j=1}^{b} \left\|v^{(j)}\right\|_2$ , b represents a number of training sample groups, and j represents a training sample weight of the $j^{th}$ training sample group. That is, the negative $L_1$ norm tends to select the samples with high confidence, namely the samples with small result errors in training. The negative $L_{2.1}$ norm is beneficial to selecting the training samples from the multiple training sample groups and embedding diversity information into the prediction model.

In all embodiments of the present disclosure, the selecting the current training sample from each training sample group based on the training sample weight to obtain a current training sample set can include:
acquiring current learning parameters, and determining a number of selected samples and sample distribution based on the current learning parameters; and selecting the current training sample from each training sample group according to the training sample weight based on the number of selected samples and the sample distribution to obtain a target current training sample set.

The current learning parameters refer to learning parameters used in the current training. The current learning parameters are used for controlling the selection of the current training sample. The number of selected samples refers to a quantity of training samples to be selected currently. The sample distribution refers to distribution of selected current training samples in each training sample group. The target current training sample set refers to a set of current training samples selected using the current learning parameters.

Specifically, the server acquires the current training sample parameters. The initial values of the current training sample parameters may be set in advance. The server uses the current learning parameters to compute the number of samples to be selected and the sample distribution in the current training. Then, the current training sample is selected from each training sample group based on the number of selected samples and the sample distribution and according to the training sample weight so as to obtain the target current training sample set.

In the above embodiment, the current learning parameters are used for further controlling the selection of the training sample so as to obtain the target current training sample set, thus the accuracy of the selected training sample is improved, furthermore, the accuracy of the prediction model obtained by training is improved, and the generalization ability of the prediction model is improved.

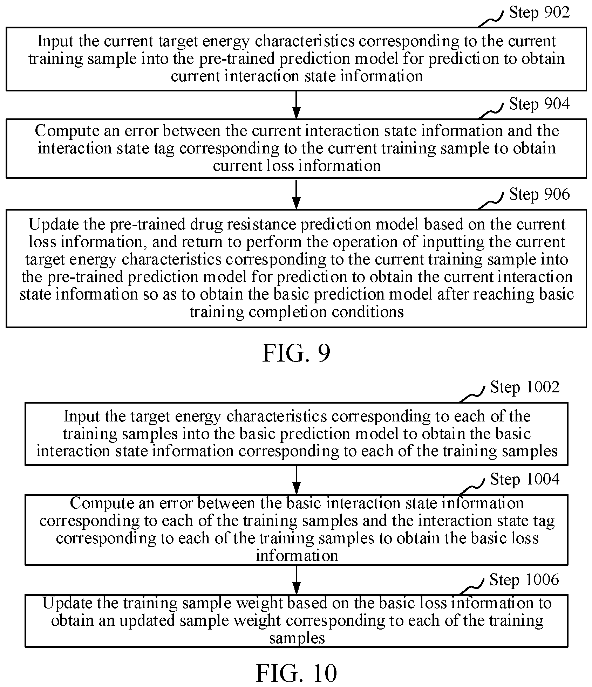

In all embodiments of the present disclosure, as shown in FIG. 9, step 206 of inputting current target energy characteristics corresponding to the current training sample into a pre-trained prediction model for basic training, and obtaining a basic prediction model after completing the basic training can include the following steps:

Step 902: Input the current target energy characteristics corresponding to the current training sample into the pre-trained prediction model for prediction to obtain current interaction state information.

The current interaction state information is used for characterizing the change of interaction between the compounds and the proteins before and after mutation in the current training sample obtained by predicting.

Specifically, the server directly treats the current target energy characteristics corresponding to the current training sample as an input of the pre-trained prediction model. The pre-trained prediction model performs prediction according to the inputted current target energy characteristics and outputs a prediction result, namely the current interaction state information.

Step 904: Compute an error between the current interaction state information and the interaction state tag corresponding to the current training sample to obtain current loss information.

The current loss information refers to an error between a prediction result corresponding to the current training sample and a real result.

Specifically, the server acquires the interaction state tag corresponding to the current training sample. The interaction state tag may be set in advance. The interaction state tag may be the change of the interaction between the compounds and the proteins before and after the mutation measured by the experiment. Then the server computes the error between the current interaction state information and the interaction state tag corresponding to the current training sample by using the preset loss function so as to obtain the current loss information.

Step 906: Update the pre-trained prediction model based on the current loss information, and return to perform the operation of inputting the current target energy characteristics corresponding to the current training sample into the pre-trained prediction model for prediction to obtain the current interaction state information so as to obtain the basic prediction model after reaching basic training completion conditions.

Specifically, the server utilizes the current loss information to reversely update the parameters in the pre-trained prediction model through a gradient descent algorithm, and returns to perform the operation of inputting the current target energy characteristics corresponding to the current training sample into the pre-trained prediction model for prediction so as to obtain the current interaction state information in an iteration way until the preset basic training iteration frequency is reached or the model parameters are no longer changed. The pre-trained prediction model obtained at the last iteration is treated as the basic prediction model.

In a specific embodiment, the optimization function corresponding to the pre-trained prediction model is shown as the following formula (2). The optimization function is a regression optimization function.

$$\min_{\pi \in \Gamma} \sum_{i=1}^{n} v_i^* L\left(g_\pi\left(x_i\right), y_i\right) \quad \text{formula (2)}$$

$v_i^*$ represents that the training sample with the training sample weight exceeding the weight threshold is selected for training. For example, in a case that the training sample weight only includes 0 and 1, the training sample with the training sample weight being 1 may be selected for training.

In the above embodiment, the training sample weight is stored to be unchanged, then the current training sample is selected to train the pre-trained prediction model to obtain the basic prediction model, and thus the accuracy of the trained basic prediction model is improved.

In all embodiments of the present disclosure, as shown in FIG. 10, step 208b of updating the training sample weight corresponding to each of the training samples based on the basic prediction model can include the following steps:

Step 1002: Input the target energy characteristics corresponding to each of the training samples into the basic prediction model to obtain the basic interaction state information corresponding to each of the training samples.

Each of the training samples refers to every training sample in the training sample set. The basic interaction state information refers to interaction state information corresponding to every training sample obtained by predicting through the basic prediction model. The interaction state information may be a relative difference value between the binding free energy of the wild type protein and the compounds and the binding free energy of the mutant type protein and the compounds.

Specifically, when the server obtains the basic prediction model by training, the parameters in the basic prediction model are kept unchanged, and the training sample weight corresponding to every training sample in the training sample set is updated. That is, the server inputs the target energy characteristic corresponding to each of the training samples into the basic prediction model to obtain the outputted basic interaction state information corresponding to each of the training samples.

Step 1004: Compute an error between the basic interaction state information corresponding to each of the training samples and the interaction state tag corresponding to each of the training samples to obtain the basic loss information.

The basic loss information refers to an error between a prediction result of the basic prediction model and a real result.

Specifically, the server computes the error of every training sample by using the preset loss function, namely computes the error between the basic interaction state information and the interaction state tag, thus obtaining the basic loss information corresponding to every training sample.

Step 1006: Update the training sample weight based on the basic loss information to obtain an updated sample weight corresponding to each of the training samples.

Specifically, the server updates every training sample weight by using the basic loss information corresponding to every training sample. The server may directly take the basic loss information corresponding to every training sample as the updated sample weight corresponding to every training sample.

In all embodiments of the present disclosure, step 1006 of updating the training sample weight based on the basic loss information to obtain an updated sample weight corresponding to each of the training samples can include:

acquiring current learning parameters, and computing an update threshold based on the current learning parameters; comparing the update threshold with the basic loss information corresponding to each of the training samples to obtain a comparison result corresponding to each of the training samples; and determining the updated sample weight corresponding to each of the training samples according to the comparison result corresponding to each of the training samples.

The update threshold refers to a threshold for updating the training sample weight.

Specifically, the server acquires the current learning parameters and determines the update threshold by using the current learning parameters. The update threshold is compared with the basic loss information corresponding to each of the training samples. In a case that the basic loss information exceeds the update threshold, it is indicated that the prediction error corresponding to the training sample is large, and the corresponding training sample weight is updated to be the first training sample weight. In a case that the basic loss information does not exceed the update threshold, it is indicated that the error is small, and the corresponding training sample weight is updated to be the second training sample weight. Then, when the current training sample is selected, the training sample corresponding to the second training sample weight is selected as the current training sample.

In all embodiments of the present disclosure, the current learning parameters can include the diversity learning parameters and the difficulty learning parameters. The computing an update threshold based on the current learning parameters includes:

acquiring each training sample group, determining a current training sample group from each training sample group, and computing a sample rank corresponding to the current training sample group; computing a weighted value based on the sample rank, and weighting the diversity learning parameters by using the weighted value to obtain a target weighted value; and computing a sum of the target weighted value and the difficulty learning parameters to obtain the update threshold.

The difficulty learning parameters refer to learning parameters for measuring the difficulty. The difficulty learning parameters are used for determining the confidence of the training sample selected in training. The diversity learning parameters are learning parameters for measuring the diversity. The diversity learning parameters are used for determining the distribution of the training samples selected during training in the training sample group. The sample rank refers to a rank of the training samples in the previous training sample group. The rank of one vector group refers to a number of vectors contained in the maximum irrelevant group of the vector group. The current training sample group refers to a training sample group needing to update the training sample weight currently.

Specifically, the server acquires each training sample group, determines the current training sample group from each training sample group, and computes the sample rank corresponding to the current training sample group; computing a weighted value based on the sample rank, and weighting the diversity learning parameters by using the weighted value to obtain a target weighted value; and computing a sum of the target weighted value and the difficulty learning parameters to obtain the update threshold corresponding to the current training sample group. In one specific embodiment, the training samples in each training sample group may be sorted in ascending order according to the basic loss information. Each sorted training sample group is obtained. The current training sample group is determined from the sorted training sample group. The update threshold corresponding to the current training sample group is computed.

In a specific embodiment, the following formula (3) may be used for updating the training sample weight corresponding to the training sample.

$$v_i^{(j)} = \begin{cases} 1, & L\left(g_{w^*}\left(x_i^{(j)}\right), y_i^j\right) < \lambda + \gamma \dfrac{1}{\sqrt{a} + \sqrt{a-1}} \\ 0, & L\left(g_{w^*}\left(x_i^{(j)}\right), y_i^j\right) \geq \lambda + \gamma \dfrac{1}{\sqrt{a} + \sqrt{a-1}} \end{cases}$$

formula (3)

$a$ represents a rank in a $j^{th}$ training sample group. $g_{w^*}\left(x_i^{(j)}\right)$ represents predicted interaction state information corresponding to an $i^{th}$ training sample in the $j^{th}$ training sample group, and $y_i^j$ represents a real interaction state tag corresponding to the $i^{th}$ training sample in the $j^{th}$ training sample group. $\lambda + \gamma \dfrac{1}{\sqrt{a} + \sqrt{a-1}}$ represents a computed update threshold. In a case that the error corresponding to the $i^{th}$ training sample in the $j^{th}$ training sample group is smaller than the update threshold, the corresponding training sample weight is updated to be 1. In a case that the error corresponding to the $i^{th}$ training sample in the $j^{th}$ training sample group is greater than or equal to the update threshold, the corresponding training sample weight is updated to be 0.

In the above embodiment, the sample weight is continuously updated, and the current training sample is reselected for training. Thus the training sample with relatively large error can be used for training in the training process, the negative influence of the training sample with the relatively large error on the training process is avoided and the accuracy of the target prediction model obtained by training is improved.

In all embodiments of the present disclosure, after updating the training sample weight corresponding to each of the training samples based on the basic prediction model, the method can further include:

acquiring current learning parameters, updating the current learning parameters according to a preset increment to obtain updated learning parameters, and treating the updated learning parameters as the current learning parameters.

Specifically, the server may preset an updating condition of the current learning parameters, for example, an increment of the current learning parameters after each weight updating is preset. Then the current learning parameters are updated according to the preset increment so as to obtain the updated learning parameters. The updated learning parameters are treated as the current learning parameters. In one embodiment, the server may further acquire the preset number of samples to be increased, update the current learning parameters through the preset number of samples to be increased so as to obtain the updated learning parameters, and treat the updated learning parameters as the current learning parameters. After the number of samples is increased, and in a case that the loss information obtained by training becomes larger, the training is completed, and the prediction model obtained by training without increasing the number of samples is treated as a finally obtained target prediction model.

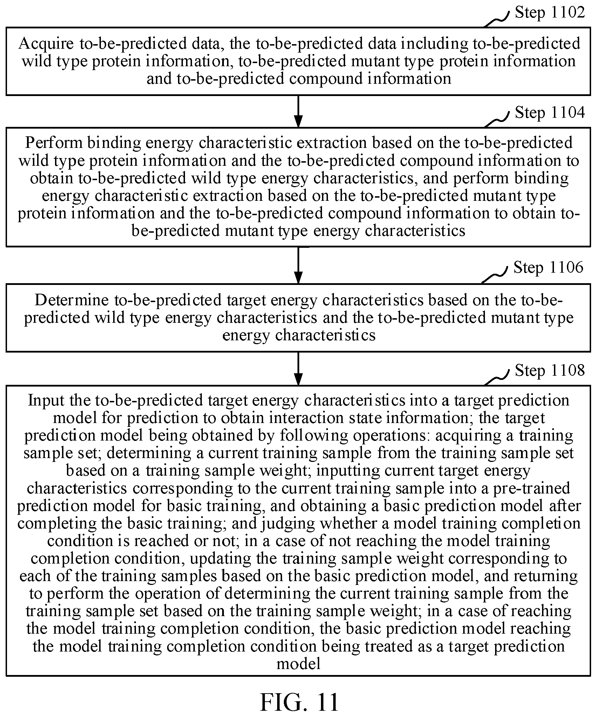

In all embodiments of the present disclosure, as shown in FIG. 11, a method for predicting data is provided. The application of this method is described by taking the server in FIG. 1 as an example. It is to be understood that this method may also be applied to the terminal and may also be applied to a system including the terminal and the server, and the method is implemented through interaction of the terminal and the server. In this embodiment, the method includes the following steps:

Step 1102: Acquire to-be-predicted data, the to-be-predicted data including to-be-predicted wild type protein information, to-be-predicted mutant type protein information and to-be-predicted compound information.

The to-be-predicted wild type protein information refers to wild type protein information requiring prediction of the interaction state information. The to-be-predicted mutant type protein information refers to mutant type protein information requiring prediction of the interaction state information. The to-be-predicted compound information refers to compound information requiring prediction of the interaction state information.

Specifically, the server may collect the to-be-predicted data from the Internet, or may also acquire the to-be-predicted data from the terminal. The server may further directly acquire the to-be-predicted data from a database. In one embodiment, the server may also acquire the to-be-predicted data transmitted by a third-party server. The third-party server may be a server providing a business service. The to-be-predicted data includes the to-be-predicted wild type protein information, the to-be-predicted mutant type protein information and the to-be-predicted compound information. In one embodiment, the server may acquire the to-be-predicted mutant type protein information and the to-be-predicted compound information from the terminal, and then acquire the to-be-predicted wild type protein information corresponding to the to-be-predicted mutant type protein information from the database so as to obtain the to-be-predicted data.

Step 1104: Perform binding energy characteristic extraction based on the to-be-predicted wild type protein information and the to-be-predicted compound information to obtain the to-be-predicted wild type energy characteristics, and perform binding energy characteristic extraction based on the to-be-predicted mutant type protein information and the to-be-predicted compound information to obtain the to-be-predicted mutant type energy characteristics.

The to-be-predicted wild type energy characteristics refer to energy characteristics obtained when the extracted to-be-predicted wild type protein information and the extracted to-be-predicted compound information interact with each other. The to-be-predicted mutant type energy characteristics refer to energy characteristics obtained when the extracted to-be-predicted mutant type protein information and the extracted to-be-predicted compound information interact with each other.

Specifically, the server performs binding energy characteristic extraction based on the to-be-predicted wild type protein information and the to-be-predicted compound information to obtain the to-be-predicted wild type energy characteristics. For example, the server may extract the structural characteristics according to a protein structure in the to-be-predicted wild type protein information and a compound structure in the to-be-predicted compound information, and then extract physicochemical property characteristics according to the physicochemical properties in the to-be-predicted wild type protein information and the physicochemical properties in the to-be-predicted compound information. The physicochemical properties are indexes for measuring chemical substance characteristics and include physical properties and chemical properties. The physical properties include melting and boiling points, states and colors at normal temperature. The chemical properties include pH values, etc. Meanwhile, the scoring function is used for computing the energy characteristics obtained when the to-be-predicted wild type protein information and the to-be-predicted compound information interact with each other, and the energy function based on mixed physical and experience potential energy is used for computing the energy characteristics, thus obtaining the to-be-predicted wild type energy characteristics. Then binding energy characteristic extraction is performed based on the to-be-predicted mutant type protein information and the to-be-predicted compound information to obtain the to-be-predicted mutant type energy characteristics. For example, the structural characteristics may be extracted according to the protein structure in the to-be-predicted mutant type protein information and the compound structure in the to-be-predicted compound information. Then physicochemical property characteristics are extracted according to physicochemical properties in the to-be-predicted mutant type protein information and physicochemical properties in the to-be-predicted compound information. Meanwhile, the scoring function is used for extracting the energy characteristics, and the energy function based on physical and experience potential energy is used for extracting the energy characteristics, thus obtaining the to-be-predicted mutant type energy characteristics.

Step 1106: Determine the to-be-predicted target energy characteristics based on the to-be-predicted wild type energy characteristics and the to-be-predicted mutant type energy characteristics.

Specifically, the server computes the difference between each characteristic value in the to-be-predicted wild type energy characteristics and the characteristic value corresponding to the to-be-predicted mutant type energy characteristics to obtain the to-be-predicted target energy characteristics.

Step 1108: Input the to-be-predicted target energy characteristics into a target prediction model for prediction to obtain interaction state information; the target prediction model being obtained by the following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and return to perform the operation of determining the current training sample from the training sample set based on the training sample weight; in a case of reaching the model training completion condition, the basic prediction model reaching the model training completion condition being treated as a target prediction model.

The target prediction model may be a model obtained by training in any embodiment of the method for training a prediction model. That is, the target prediction model may acquire the training sample set, and determine the current training sample from the training sample set based on the training sample weight; input the current target energy characteristics corresponding to the current training sample into the pre-trained prediction model for basic training, and obtain the basic prediction model after completing the basic training; and update the training sample weight corresponding to each of the training samples based on the basic prediction model, and return to perform the operation of determining the current training sample from the training sample set based on the training sample weight until completing the model training.

Specifically, the server inputs the to-be-predicted target energy characteristics into the target prediction model for prediction so as to obtain outputted interaction state information. In a specific embodiment, the interaction state information refers to a relative difference value between the binding free energy of the to-be-predicted mutant type protein and the to-be-predicted compound and the binding free energy of the to-be-predicted wild type protein and the to-be-predicted compound. Then the relative difference value of the binding free energy is compared with a drug resistance threshold. In a case that the relative difference value of the binding free energy exceeds the drug resistance threshold, it is indicated that the to-be-predicted mutant type protein has drug resistance and cannot be used continuously. In a case that the relative difference value of the binding free energy does not exceed the drug resistance threshold, it is indicated that the to-be-predicted mutant type protein does not have drug resistance and can still be used normally. In some embodiments, when a protein corresponding to a target of a disease is mutated, in order to predict whether a drug still works for the target, the target prediction model can be configured to perform the prediction according to the protein information (e. g.,including wild type protein information and mutant type protein information corresponding to the target) and the compound information (e. g., a small-molecule drug corresponding to the protein information of the target) of a drug to obtain predicted interaction state information. In some embodiments, the interaction state information refers to a relative difference value between the binding free energy of the to-be-predicted mutant type protein and the to-be-predicted compound and the binding free energy of the to-be-predicted wild type protein and the to-be-predicted compound. When the relative difference value represented by the interaction state information exceeds a drug resistance threshold, it is determined that the mutant type protein corresponding to the mutant type protein information is resistant to the described drug, i. e. the drug does not act on the target; if the relative difference value represented by the described interaction state information does not exceed the drug resistance threshold, it is determined that the mutant type protein corresponding to the mutant type protein information does not produce resistance to the described drug, i. e. the drug still acts on the target.

According to the method and apparatus for predicting data, the computer device and the storage medium, the to-be-predicted data is acquired, the to-be-predicted target energy characteristics are determined, and the to-be-predicted target energy characteristics are inputted into the target prediction model for prediction to obtain the interaction state information; the target prediction model determines the current training sample from the training sample set based on the training sample weight by acquiring the training sample set; the current target energy characteristics corresponding to the current training sample are inputted into the pre-trained prediction model for basic training, and the basic prediction model is obtained after completing the basic training; and the training sample weight corresponding to each of the training samples is updated based on the basic prediction model, and the operation of determining the current training sample from the training sample set is performed based on the training sample weight until completing the model training; that is, the interaction state information is obtained by predicting through the target prediction model; and the target prediction model obtained by training can improve the prediction accuracy so that the accuracy of the obtained interaction state information can be improved.

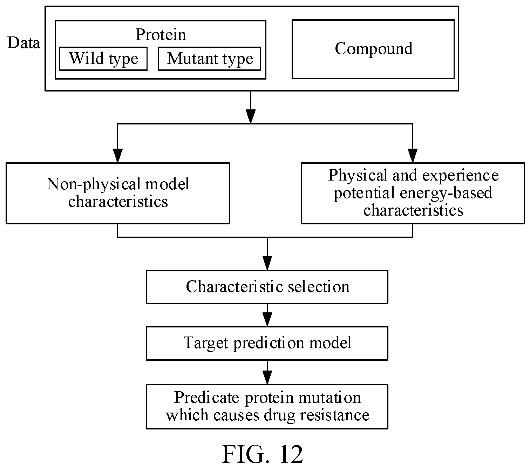

This application further provides an application scenario, and the foregoing method for predicting data is applied to the application scenario. FIG. 12 is a schematic flow diagram of an application scene of a method for predicting data. Specifically, the server acquires the to-be-predicted data transmitted by a terminal in an application scene for predicting drug resistance caused by targeted protein mutation. The to-be-predicted data includes two different types of targeted protein information, including the wild type protein information, the mutant type protein information and the compound information. Then the wild type protein information, the mutant type protein information and the compound information are used for extracting characteristics with reference value for predicting the affinity of protein after mutation. The characteristics include characteristics of the non-physical model and characteristics based on physical and experience potential energy. The characteristics of the non-physical model are, for example, the crystal protein-ligand structure characteristics, physicochemical property characteristics of ligands and residues, some energy characteristics obtained by computing by the experience or descriptor-based scoring function, etc. Then the characteristics based on physical and experience potential energy are energy characteristics computed by using the modeling program Rosetta based on the mixed physical and experience potential energy. Then characteristic selection is carried out. Namely, corresponding characteristics are selected from the extracted characteristics through target energy characteristics obtained by characteristic selection during training so as to obtain the to-be-predicted target energy characteristics, and the to-be-predicted target energy characteristics are inputted into the target prediction model for prediction to obtain the difference value of predicted binding free energy. The difference value of the binding free energy is compared with the drug resistance threshold. In a case that the difference value of the binding free energy exceeds the drug resistance threshold, it is indicated that the protein mutation is a protein mutation which may cause drug resistance. In a case that the difference value of the binding free energy does not exceed the drug resistance threshold, it is indicated that the protein mutation is a protein mutation which does not cause drug resistance. And at this time, the prediction result is transmitted to the terminal for display.

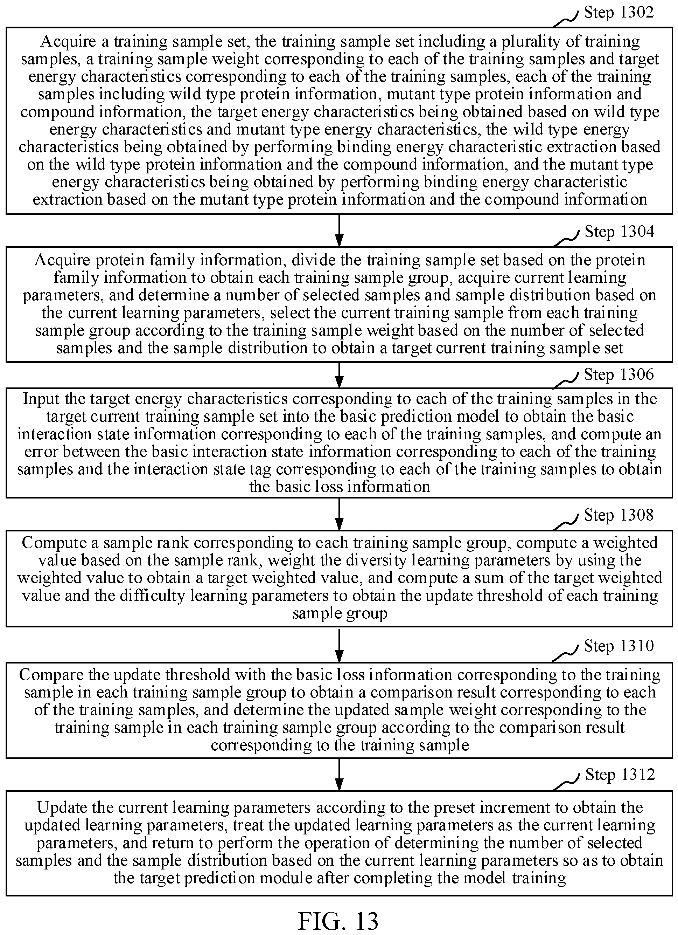

In a specific embodiment, as shown in FIG. 13, a method for training a prediction model is provided. The method specifically includes the following steps:

Step 1302: Acquire a training sample set, the training sample set including a plurality of training samples, a training sample weight corresponding to each of the training samples and the target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information.

Step 1304: Acquire protein family information, divide the training sample set based on the protein family information to obtain each training sample group, acquire current learning parameters, and determine a number of selected samples and sample distribution based on the current learning parameters. The current training sample is selected from each training sample group according to the training sample weight based on the number of selected samples and the sample distribution to obtain a target current training sample set.

Step 1306: Input the target energy characteristics corresponding to each of the training samples in the target current training sample set into the basic prediction model to obtain the basic interaction state information corresponding to each of the training samples, and compute an error between the basic interaction state information corresponding to each of the training samples and the interaction state tag corresponding to each of the training samples to obtain the basic loss information.

Step 1308: Compute a sample rank corresponding to each training sample group. A weighted value is computed based on the sample rank. The diversity learning parameters are weighted by using the weighted value to obtain a target weighted value. A sum of the target weighted value and the difficulty learning parameters is computed to obtain the update threshold of each training sample group.

Step 1310: Compare the update threshold with the basic loss information corresponding to the training sample in each training sample group to obtain a comparison result corresponding to each of the training samples, and determine the updated sample weight corresponding to the training sample in each training sample group according to the comparison result corresponding to the training sample.

Step 1312: Update the current learning parameters according to the preset increment to obtain the updated learning parameters, treat the updated learning parameters as the current learning parameters, and return to perform the operation of determining the number of selected samples and the sample distribution based on the current learning parameters so as to obtain the target prediction module after completing the model training.

This application further provides an application scenario, and the foregoing method for predicting a prediction model is applied to the application scenario. Specifically:

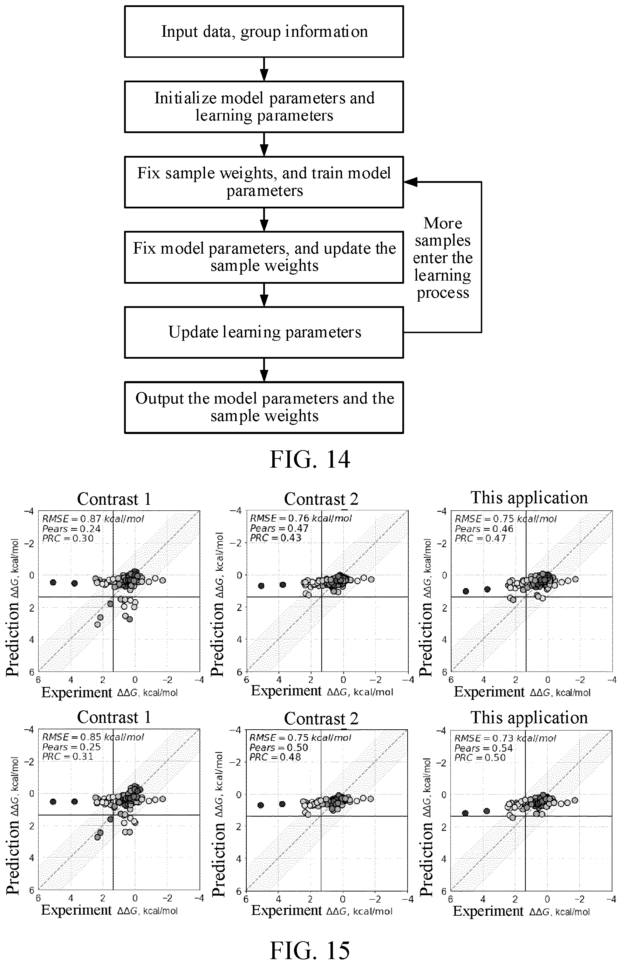

FIG. 14 is a schematic flow diagram of a method for training a prediction model, specifically including the following steps:

The input data and the training sample group information are acquired. The input data includes each of the training samples, the corresponding training sample weight is 0 or 1, and the training sample group information indicates a training sample group to which the training sample in the input data belongs. At this time, the model parameters and learning parameters of the prediction model are initialized.

Then the training sample weight corresponding to the training sample is fixed, the parameters of the model are trained, that is, the training sample with the training sample weight of 1 is selected according to the initialized learning parameters, and thus the current training sample is obtained. The current target energy characteristics corresponding to the current training sample are extracted, and the current target energy characteristics are inputted into the initialized prediction model for basic training. The basic prediction model is obtained after completing the basic training.

Then the parameters of the basic prediction model are fixed, and the sample weight is updated. That is, the training sample weight corresponding to each of the training samples is updated by using the formula (3), and thus the updated sample weight is obtained.

At this time, the initialized learning parameters are further updated. Then the operation of fixing the training sample weight corresponding to the training sample and training the parameters of the model is performed again in a continuous iteration way, and the model parameters of the prediction model and the training sample weight are outputted after completing the model training, thus obtaining the target prediction model.

In this embodiment, the target prediction model obtained by training is subjected to a comparison test. Specifically, a drug resistance standard data set Platinum (Platinum is a database widely collecting drug resistance information and is developed for studying and understanding the influence of missense mutation on the interaction of the ligands and a proteome) and TKI are used for training and testing. The data set Platinum is used for training to obtain the target prediction model, and then the data set TKI is used for testing. By adopting RDKit (RDKit is an open source kit for chemical informatics, and is used for performing compound descriptor generation, fingerprint generation, compound structural similarity computing, 2D and 3D molecules display, etc. through a machine learning method based on the compound 2D and 3D molecule operation), Biopython (Biopython provides an online resource library for developers using and studying bioinformatics), FoldX (computing protein binding free energy), PLIP (being an analysis tool for protein-ligand non-covalent interaction), AutoDock (being open source molecular simulation software, and most mainly applied to performing ligand-protein molecule docking) and other non-physical model tools, the characteristics corresponding to the affinity change after protein mutation are predicted are generated. The modeling program Rosetta based on mixed physical and experience potential energy is used for computing the energy characteristics. Then characteristic selection is carried out to obtain finally selected characteristics. The details are shown in table 1 below, which is a finally selected characteristic number table.

Table 1 Characteristic number table

| Data set | Number of samples | Non-physical model characteristics | Physical and experience potential energy characteristics | Total number of characteristics |
|---|---|---|---|---|
| Platinum | 484 | 129 | 19 | 148 |
| TKI | 144 | 129 | 19 | 148 |

At this time, the target prediction model obtained by training is subjected to comparison test, and the test result is shown in FIG. 15. FIG. 15 shows a scatter diagram of the $\Delta\Delta G$ value measured by the experiment and obtained by prediction. The $\Delta\Delta G$ is the relative difference value of the binding free energy of the ligand and the receptor, that is, the difference value of the corresponding binding free energy when the protein before and after mutation is bound with the compound. The first row in FIG. 15 is a schematic result diagram of predicting the relative difference value of the binding free energy by using only the non-physical model characteristics. The second row in FIG. 15 is a schematic result diagram of predicting the relative difference value of the binding free energy by using the non-physical model characteristics and the physical and experience potential energy characteristics together. The first column is a scatter diagram of the relative difference value of the binding free energy obtained by testing through the related technology 1. The second column is a scatter diagram of the relative difference value of the binding free energy obtained by testing through the related technology 2. The third column is a scatter diagram of the relative difference value of the binding free energy obtained by testing through the technical scheme of this application. Root mean square error (RMSE), Pearson correlation coefficient (Pearson) (which is used for measuring whether two data sets are on the same line, as well as measuring a linear relationship between the fixed distance variables) and AUPRC (precision recall curve with decreasing area under curve) are treated as evaluation indexes. The mean, minimum and maximum values of RMSE, Pearson and AUPRC indexes are computed respectively, and the obtained results are shown in Table 2.

Table 2 Evaluation index table

| Characteristics | Method | RMSE (kcal/mol) | Pearson | AUPRC |
|---|---|---|---|---|
| Non-physical model characteristics | Related technology 1 | 0.87 (0.85, 0.90) | 0.24 (0.20, 0.27) | 0.29 (0.26, 0.33) |
| | Related technology 2 | 0.76 (0.75, 0.77) | 0.47 (0.38, 0.50) | 0.43 (0.33, 0.50) |
| | This application | 0.75 (0.73, 0.76) | 0.46 (0.40, 0.49) | 0.47 (0.38, 0.48) |
| All characteristics | Related technology 1 | 0.85 (0.84, 0.88) | 0.25 (0.21, 0.29) | 0.31 (0.26, 0.33) |
| | Related technology 2 | 0.75 (0.75, 0.77) | 0.50 (0.38, 0.54) | 0.48 (0.34, 0.52) |
| | This application | 0.73 (0.72, 0.74) | 0.54 (0.47, 0.56) | 0.50 (0.43, 0.52) |

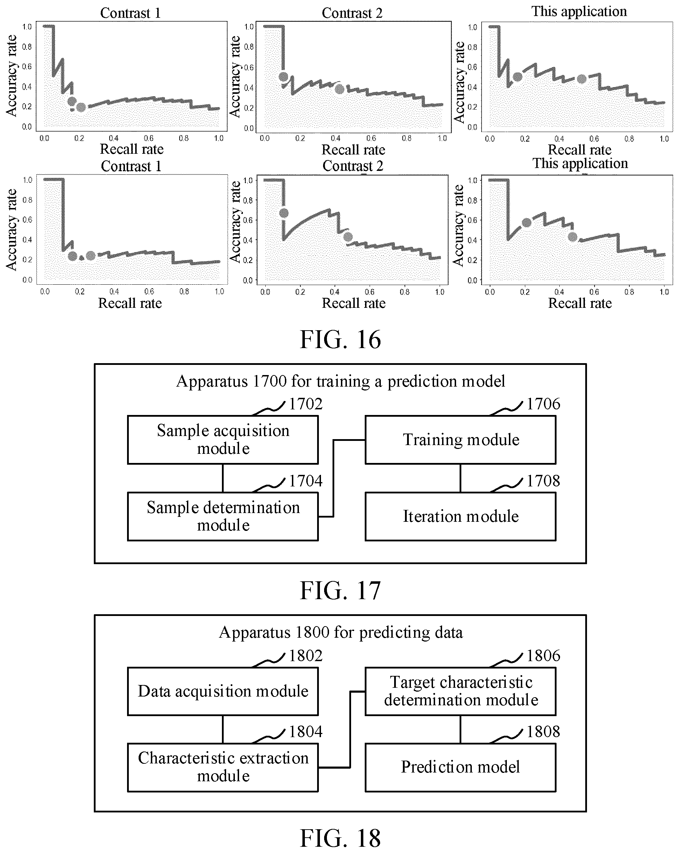

In all characteristics, the average value of the RMSE (the smaller the better) index in this application is 0.73, the minimum value is 0.72, the maximum value is 0.74, and the average error is obviously smaller than other related art. The Pearson (the larger the better) index in this application is also obviously superior to other related technologies. The AUPRC index in this application is also superior to other related technologies. Therefore, compared with the related technologies, the prediction accuracy in this application is obviously improved. Further, FIG. 16 is a schematic diagram of the AUPRC index in the comparison test result. A first circle from left to right in each curve represents that when $\Delta\Delta G >$ 1.36 kcal/mol is treated as a threshold, and the test sample obtains a corresponding drug resistance result, the precision and the recall rate corresponding to the drug resistance result are predicted. The second circle from left to right in each curve represents that when the previous 15% $\Delta\Delta G$ test sample is treated as the drug resistance division result, the precision and the recall rate corresponding to the drug resistance result are predicted. Obviously, the technical solution of this application can obviously improve the performance of dividing the sample with drug resistance and the sample without drug resistance.

It is to be understood that, steps in flowcharts of FIG. 2 to FIG. 14 are displayed in sequence based on indication of arrows, but the steps are not necessarily performed in sequence based on a sequence indicated by the arrows. Unless otherwise explicitly specified in this specification, execution of the steps is not strictly limited, and the steps may be performed in other sequences. In addition, at least some steps in FIG. 2 to FIG. 14 may include a plurality of steps or a plurality of stages, and these steps or stages are not necessarily performed at a same time instant, but may be performed at different time instants. The steps or stages are not necessarily performed in sequence, but may be performed by turn or alternately with other steps or at least part of steps or stages in other steps.

In all embodiments of the present disclosure, as shown in FIG. 17, an apparatus 1700 for training a prediction model is provided. The apparatus may adopt a software module or a hardware module, or a combination of the software module and the hardware module is treated as a part of the computer device. The apparatus specifically includes a sample acquisition module 1702, a sample determination module 1704, a training module 1706 and an iteration module 1708.

The sample acquisition module 1702 is configured to acquire a training sample set. The training sample set includes a plurality of training samples, a training sample weight corresponding to each of the training samples and target energy characteristics corresponding to each of the training samples. Each of the training samples includes wild type protein information, mutant type protein information and compound information. The target energy characteristics are obtained based on wild type energy characteristics and mutant type energy characteristics. The wild type energy characteristics are obtained by performing binding energy characteristic extraction based on the wild type protein information and the compound information. The mutant type energy characteristics are obtained by performing binding energy characteristic extraction based on the mutant type protein information and the compound information.

The sample determination module 1704 is configured to determine a current training sample from the training sample set based on the training sample weight.

The training module 1706 is configured to input current target energy characteristics corresponding to the current training sample into a pre-trained prediction model for basic training, and obtain a basic prediction model after completing the basic training.

The iteration module 1708 is configured to judge whether a model training completion condition is reached or not; in a case of not reaching the model training completion condition, update the training sample weight corresponding to each of the training samples based on the basic prediction model, and return to perform the operation of determining the current training sample from the training sample set based on the training sample weight; in a case of reaching the model training completion condition, treat the basic prediction model reaching the model training completion condition as a target prediction model. The target prediction model is configured to predict interaction state information corresponding to the inputted protein information and the inputted compound information.

In all embodiments of the present disclosure, the apparatus 1700 for training a prediction model can further include:

a pre-training module, configured to acquire each of the training samples, each of the training samples including the wild type protein information, the mutant type protein information and the compound information; perform binding initial energy characteristic extraction based on the wild type protein information and the compound information to obtain wild type initial energy characteristics; perform binding initial energy characteristic extraction based on the mutant type protein information and the compound information to obtain mutant type initial energy characteristics, and determine target initial energy characteristics corresponding to each of the training samples based on the wild type initial energy characteristics and the mutant type initial energy characteristics; input the target initial energy characteristics corresponding to each of the training samples into an initial prediction model for prediction to obtain initial interaction state information corresponding to each of the training samples, the initial prediction model being established by using a random forest algorithm; perform loss computation based on the initial interaction state information corresponding to each of the training samples and the interaction state tag corresponding to each of the training samples to obtain initial loss information corresponding to each of the training samples; update the initial prediction model based on the initial loss information, and return to perform the operation of inputting the target energy characteristics corresponding to each of the training samples into the initial prediction model for prediction until completing the pre-training, thus obtaining the pre-trained prediction model and characteristic importance corresponding to the target initial energy characteristics; and determine the training sample weight corresponding to each of the training samples based on the loss information corresponding to each of the training samples in a case of completing pre-training, and select the target energy characteristics from the target initial energy characteristics based on the characteristic importance.

In all embodiments of the present disclosure, the pre-training module can be further configured to input the target initial energy characteristics corresponding to each of the training samples into the initial prediction model; and the initial prediction model can be configured to treat the target initial energy characteristics corresponding to each of the training samples as a current to-be-divided set, and compute initial characteristic importance corresponding to the target initial energy characteristics; determine initial division characteristics from the target initial energy characteristics based on the initial characteristic importance; divide the target initial energy characteristics corresponding to each of the training samples based on the initial division characteristics so as to obtain each division result, the division result including target initial energy characteristics corresponding to each division sample; and treat each division result as the current to-be-divided set, and return to perform the operation of computing the initial characteristic importance corresponding to the target initial energy characteristics for iteration until completing division, thus obtaining initial interaction state information corresponding to each of the training samples.

In all embodiments of the present disclosure, the sample acquisition module 1702 can be further configured to acquire confidence corresponding to each of the training samples, and determine the training sample weight corresponding to each of the training samples based on the confidence.

In all embodiments of the present disclosure, the sample acquisition module 1702 can be further configured to perform binding energy characteristic extraction based on the wild type protein information and the compound information to obtain the wild type energy characteristics; perform binding energy characteristic extraction based on the mutant type protein information and the compound information to obtain the mutant type energy characteristics; and compute a difference between the wild type energy characteristics and the mutant type energy characteristics to obtain the target energy characteristics.

In all embodiments of the present disclosure, the wild type energy characteristics can include first wild type energy characteristics and second wild type energy characteristics. The sample acquisition module 1702 is further configured to perform binding energy characteristic extraction by a non-physical scoring function based on wild type protein information and compound information to obtain the first wild type energy characteristics; perform binding energy characteristic extraction by a physical function based on the wild type protein information and the compound information to obtain the second wild type energy characteristics; and perform fusing based on the first wild type energy characteristics and the second wild type energy characteristics to obtain the wild type energy characteristics.

In all embodiments of the present disclosure, the mutant type energy characteristics can include first mutant type energy characteristics and second mutant type energy characteristics. The sample acquisition module 1702 is further configured to perform binding energy characteristic extraction by a non-physical function based on the mutant type protein information and the compound information to obtain the first mutant type energy characteristics; perform binding energy characteristic extraction by a physical function based on the mutant type protein information and the compound information to obtain the second mutant type energy characteristics; and perform fusing based on the first mutant type energy characteristics and the second mutant type energy characteristics to obtain the mutant type energy characteristics.

In all embodiments of the present disclosure, the sample determination module 1704 can be further configured to acquire protein family information, and divide the training sample set based on the protein family information to obtain each training sample group; and select the current training sample from each training sample group based on the training sample weight to obtain a current training sample set.

The training module 1706 is further configured to input the current target energy characteristics corresponding to each current training sample in the current training sample set into the pre-trained prediction model for basic training, and obtain a target basic prediction model after completing the basic training.

In all embodiments of the present disclosure, the sample determination module 1704 can be further configured to acquire current learning parameters, and determine a number of selected samples and sample distribution based on the current learning parameters; and select the current training sample from each training sample group according to the training sample weight based on the number of selected samples and the sample distribution to obtain a target current training sample set.

In all embodiments of the present disclosure, the training module 1706 can be further configured to input the current target energy characteristics corresponding to the current training sample into the pre-trained prediction model for prediction to obtain current interaction state information; compute an error between the current interaction state information and the interaction state tag corresponding to the current training sample to obtain the current loss information; and update the pre-trained prediction model based on the current loss information, and return to perform the operation of inputting the current target energy characteristics corresponding to the current training sample into the pre-trained prediction model for prediction to obtain the current interaction state information so as to obtain the basic prediction model after reaching the basic training completion conditions.

In all embodiments of the present disclosure, the iteration module 1708 can be further configured to input the target energy characteristics corresponding to each of the training samples into the basic prediction model to obtain the basic interaction state information corresponding to each of the training samples; compute an error between the basic interaction state information corresponding to each of the training samples and the interaction state tag corresponding to each of the training samples to obtain basic loss information; and update the training sample weight based on the basic loss information to obtain an updated sample weight corresponding to each of the training samples.

In all embodiments of the present disclosure, the iteration module 1708 can be further configured to acquire current learning parameters, and compute an update threshold based on the current learning parameters; compare the update threshold with the basic loss information corresponding to each of the training samples to obtain a comparison result corresponding to each of the training samples; and determine the updated sample weight corresponding to each of the training samples according to the comparison result corresponding to each of the training samples.

In all embodiments of the present disclosure, the current learning parameters can include diversity learning parameters and difficulty learning parameters. The iteration module 1708 is further configured to acquire each training sample group, determine a current training sample group from each training sample group, and compute a sample rank corresponding to the current training sample group; compute a weighted value based on the sample rank, and weight the diversity learning parameters by using the weighted value to obtain a target weighted value; and compute a sum of the target weighted value and the difficulty learning parameters to obtain the update threshold.

In all embodiments of the present disclosure, the iteration module 1708 can acquire current learning parameters, update the current learning parameters according to a preset increment to obtain updated learning parameters, and treat the updated learning parameters as the current learning parameters.

In all embodiments of the present disclosure, as shown in FIG. 18, an apparatus 1800 for predicting data is provided. The apparatus may adopt a software module or a hardware module, or a combination of the software module and the hardware module is treated a part of the computer device. The apparatus specifically includes a data acquisition module 1802, a characteristic extraction module 1804, a target characteristic determination module 1806 and a prediction module 1808.

The data acquisition module 1802 is configured to acquire to-be-predicted data, the to-be-predicted data including to-be-predicted wild type protein information, to-be-predicted mutant type protein information and to-be-predicted compound information.

The characteristic extraction module 1804 is configured to perform binding energy characteristic extraction based on the to-be-predicted wild type protein information and the to-be-predicted compound information to obtain to-be-predicted wild type energy characteristics, and perform binding energy characteristic extraction based on the to-be-predicted mutant type protein information and the to-be-predicted compound information to obtain to-be-predicted mutant type energy characteristics.

The target characteristic determination module 1806 is configured to determine to-be-predicted target energy characteristics based on the to-be-predicted wild type energy characteristics and the to-be-predicted mutant type energy characteristics.

The prediction module 1808 is configured to input the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model.

For specific definitions on the apparatus for training a prediction model and the apparatus for predicting data, reference may be made to the above definitions on the method for training a prediction model and the method for predicting data, no more description is made herein. The modules in the foregoing apparatus for predicting data may be implemented entirely or partially by software, hardware, or a combination thereof. The foregoing modules may be built in or independent of a processor of a computer device in a hardware form, or may be stored in a memory of the computer device in a software form, so that the processor invokes and performs an operation corresponding to each of the foregoing modules.

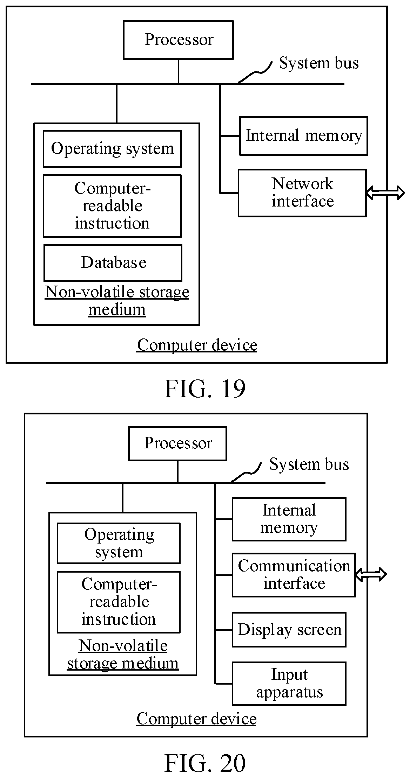

In all embodiments of the present disclosure, a computer device is provided. The computer device may be a server, and an internal structure diagram thereof may be shown in FIG. 19. The computer device includes a processor, a memory, and a network interface that are connected by using a system bus. The processor of the computer device is configured to provide computing and control capabilities. The memory of the computer device includes a non-volatile storage medium and an internal memory. The non-volatile storage medium stores an operating system, computer-readable instructions, and a database. The internal memory provides an environment for running of the operating system and the computer-readable instructions in the non-volatile storage medium. The database of the computer device is configured to store training sample data and to-be-predicted data. The network interface of the computer device is configured to communicate with an external terminal through a network connection. The computer-readable instructions are executed by the processor to implement a method for training a prediction model or a method for predicting data.

In all embodiments of the present disclosure, a computer device is provided. The computer device may be a terminal, and an internal structure diagram thereof may be shown in FIG. 20. The computer device includes a processor, a memory, a communication interface, a display screen, and an input apparatus that are connected by using a system bus. The processor of the computer device is configured to provide computing and control capabilities. The memory of the computer device includes a non-volatile storage medium and an internal memory. The non-volatile storage medium stores an operating system and computer-readable instructions. The internal memory provides an environment for running of the operating system and the computer-readable instructions in the non-volatile storage medium. The communication interface of the computer device is configured to communicate with an external terminal in a wired or a wireless manner, and the wireless manner can be implemented by using WIFI, an operator network, NFC, or other technologies. The computer-readable instructions are executed by the processor to implement a method for training a prediction model and a method for predicting data. The display screen of the computer device may be a liquid crystal display screen or an electronic ink display screen. The input apparatus of the computer device may be a touch layer covering the display screen, or may be a key, a trackball, or a touch pad disposed on a housing of the computer device, or may be an external keyboard, a touch pad, a mouse, or the like.

A person skilled in the art may understand that, the structure shown in FIG. 19 and 20 is only a block diagram of a part of a structure related to a solution of this application and does not limit the computer device to which the solution of this application is applied. Specifically, the computer device may include more or fewer components than those in the drawings, or some components are combined, or a different component deployment is used.

In all embodiments of the present disclosure, a computer device is further provided, including a memory and a processor, the memory storing computer-readable instructions, the processor, when executing the computer-readable instructions, implementing the steps in the foregoing method embodiments.

In all embodiments of the present disclosure, a computer-readable storage medium is provided, storing computer-readable instructions, the computer-readable instructions, when executed by a processor, implementing the steps in the foregoing method embodiments.

In all embodiments of the present disclosure, a computer program product or a computer program is provided. The computer program product or the computer program includes computer instructions, the computer instructions being stored in a computer-readable storage medium. The processor of the computer device reads the computer instructions from the computer-readable storage medium, and the processor executes the computer instructions, to cause the computer device to perform the steps in the above method embodiments.

A person of ordinary skill in the art may understand that all or some of procedures of the method in the foregoing embodiments may be implemented by a computer program instructing relevant hardware. The program may be stored in a non-volatile computer-readable storage medium. When the program is executed, the procedures of the foregoing method embodiments may be implemented. Any reference to a memory, a storage, a database, or another medium used in the embodiments provided in this application may include at least one of a non-volatile memory and a volatile memory. The non-volatile memory may include a read-only memory (ROM), a magnetic tape, a floppy disk, a flash memory, an optical memory, and the like. The volatile memory may include a random access memory (RAM) or an external cache. For the purpose of description instead of limitation, the RAM is available in a plurality of forms, such as a static RAM (SRAM) or a dynamic RAM (DRAM).

The technical features in the foregoing embodiments may be randomly combined. For concise description, not all possible combinations of the technical features in the embodiment are described. However, provided that combinations of the technical features do not conflict with each other, the combinations of the technical features are considered as falling within the scope recorded in this specification.

The foregoing embodiments only describe several implementations of this application specifically and in detail, but cannot be construed as a limitation to the patent scope of this application. A person of ordinary skill in the art may make various changes and improvements without departing from the ideas of this application, which shall all fall within the protection scope of this application. Therefore, the protection scope of this patent application is subject to the protection scope of the appended claims.

**Claims**

1. A method for training a prediction model, performed by a computer device, comprising:

acquiring a training sample set, the training sample set comprising a plurality of training samples, a training sample weight corresponding to each of the training samples and target energy characteristics corresponding to each of the training samples, each of the training samples comprising wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information; determining a current training sample from the training sample set based on the training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model, the target prediction model being configured to predict interaction state information corresponding to the inputted protein information and the inputted compound information.

2. The method according to claim 1, wherein before acquiring the training sample set, the method further comprises:

acquiring each of the training samples and an interaction state tag corresponding to each of the training samples, each of the training samples comprising the wild type protein information, the mutant type protein information and the compound information; performing binding initial energy characteristic extraction based on the wild type protein information and the compound information to obtain wild type initial energy characteristics; performing binding initial energy characteristic extraction based on the mutant type protein information and the compound information to obtain mutant type initial energy characteristics, and determining target initial energy characteristics corresponding to each of the training samples based on the wild type initial energy characteristics and the mutant type initial energy characteristics; inputting the target initial energy characteristics corresponding to each of the training samples into an initial prediction model for prediction to obtain initial interaction state information corresponding to each of the training samples, the initial prediction model being established by using a random forest algorithm; performing loss computation based on the initial interaction state information corresponding to each of the training samples and the interaction state tag corresponding to each of the training samples to obtain initial loss information corresponding to each of the training samples; updating the initial prediction model based on the initial loss information, and returning to perform the operation of inputting the target energy characteristics corresponding to each of the training samples into the initial prediction model for prediction until completing the pre-training, thus obtaining characteristic importance corresponding to the pre-trained prediction model and the target initial energy characteristics; and determining the training sample weight corresponding to each of the training samples based on the loss information corresponding to each of the training samples in case of completing pre-training, and selecting the target energy characteristics from the target initial energy characteristics based on the characteristic importance.

3. The method according to claim 2, wherein the inputting the target initial energy characteristics corresponding to each of the training samples into an initial prediction model for prediction to obtain initial interaction state information corresponding to each of the training samples, the initial prediction model being established by using a random forest algorithm comprises:

inputting the target initial energy characteristics corresponding to each of the training samples into the initial prediction model; and treating, by the initial prediction model, the target initial energy characteristics corresponding to each of the training samples as a current to-be-divided set, and computing initial characteristic importance corresponding to the target initial energy characteristics; determining initial division characteristics from the target initial energy characteristics based on the initial characteristic importance; dividing the target initial energy characteristics corresponding to each of the training samples based on the initial division characteristics so as to obtain each division result, the division result comprising target initial energy characteristics corresponding to each division sample; and treating each division result as the current to-be-divided set, and returning to perform the operation of computing the initial characteristic importance corresponding to the target initial energy characteristics for iteration until completing division, thus obtaining initial interaction state information corresponding to each of the training samples.

4. The method according to claim 1, wherein the acquiring a training sample set, the training sample set comprising the training sample weight corresponding to each of the training samples comprises:
acquiring confidence corresponding to each of the training samples, and determining the training sample weight corresponding to each of the training samples based on the confidence.

5. The method according to claim 1, wherein the acquiring the training sample set, the training sample set comprising the target energy characteristics corresponding to each of the training samples comprises:

   performing binding energy characteristic extraction based on the wild type protein information and the compound information to obtain the wild type energy characteristics;
   performing binding energy characteristic extraction based on the mutant type protein information and the compound information to obtain the mutant type energy characteristics; and
   computing a difference between the wild type energy characteristics and the mutant type energy characteristics to obtain the target energy characteristics.

6. The method according to claim 5, wherein the wild type energy characteristics comprise first wild type energy characteristics and second wild type energy characteristics;
   the performing binding energy characteristic extraction based on the wild type protein information and the compound information to obtain the wild type energy characteristics comprises:

   performing binding energy characteristic extraction by a non-physical scoring function based on the wild type protein information and the compound information to obtain the first wild type energy characteristics;
   performing binding energy characteristic extraction by a physical function based on the wild type protein information and the compound information to obtain the second wild type energy characteristics; and
   performing fusing based on the first wild type energy characteristics and the second wild type energy characteristics to obtain the wild type energy characteristics.

7. The method according to claim 5, wherein the mutant type energy characteristics comprise first mutant type energy characteristics and second mutant type energy characteristics;
   the performing binding energy characteristic extraction based on the mutant type protein information and the compound information to obtain the mutant type energy characteristics comprises:

   performing binding energy characteristic extraction by a non-physical function based on the mutant type protein information and the compound information to obtain the first mutant type energy characteristics;
   performing binding energy characteristic extraction by a physical function based on the mutant type protein information and the compound information to obtain the second mutant type energy characteristics; and
   performing fusing based on the first mutant type energy characteristics and the second mutant type energy characteristics to obtain the mutant type energy characteristics.

8. The method according to claim 1, wherein the determining the current training sample from the training sample set based on the training sample weight comprises:

   acquiring protein family information, and dividing the training sample set based on the protein family information to obtain each training sample group; and
   selecting the current training sample from each training sample group based on the training sample weight to obtain a current training sample set; and
   the inputting current target energy characteristics corresponding to the current training sample into a pre-trained prediction model for basic training, and obtaining a basic prediction model after completing the basic training comprises:
   inputting the current target energy characteristics corresponding to each current training sample in the current training sample set into the pre-trained prediction model for basic training, and obtaining a target basic prediction model after completing the basic training.

**9.** The method according to claim 8, wherein the selecting the current training sample from each training sample group based on the training sample weight to obtain a current training sample set comprises:

acquiring current learning parameters, and determining a number of selected samples and sample distribution based on the current learning parameters; and
selecting the current training sample from each training sample group according to the training sample weight based on the number of selected samples and the sample distribution to obtain a target current training sample set.

**10.** The method according to claim 1, wherein the inputting current target energy characteristics corresponding to the current training sample into a pre-trained prediction model for basic training, and obtaining a basic prediction model after completing the basic training comprises:

inputting the current target energy characteristics corresponding to the current training sample into the pre-trained prediction model for prediction to obtain current interaction state information;
computing an error between the current interaction state information and the interaction state tag corresponding to the current training sample to obtain current loss information; and
updating the pre-trained prediction model based on the current loss information, and returning to perform the operation of inputting the current target energy characteristics corresponding to the current training sample into the pre-trained prediction model for prediction to obtain the current interaction state information so as to obtain the basic prediction model after reaching basic training completion conditions.

**11.** The method according to claim 1, wherein the updating the training sample weight corresponding to each of the training samples based on the basic prediction model comprises:

inputting the target energy characteristics corresponding to each of the training samples into the basic prediction model to obtain the basic interaction state information corresponding to each of the training samples;
computing an error between the basic interaction state information corresponding to each of the training samples and the interaction state tag corresponding to each of the training samples to obtain basic loss information; and
updating the training sample weight based on the basic loss information to obtain an updated sample weight corresponding to each of the training samples.

**12.** The method according to claim 11, wherein the updating the training sample weight based on the basic loss information to obtain an updated sample weight corresponding to each of the training samples comprises:

acquiring current learning parameters, and computing an update threshold based on the current learning parameters;
comparing the update threshold with the basic loss information corresponding to each of the training samples to obtain a comparison result corresponding to each of the training samples; and
determining the updated sample weight corresponding to each of the training samples according to the comparison result corresponding to each of the training samples.

**13.** The method according to claim 12, wherein the current learning parameters comprise diversity learning parameters and difficulty learning parameters; and
the computing an update threshold based on the current learning parameters comprises:

acquiring each training sample group, determining a current training sample group from each training sample group, and computing a sample rank corresponding to the current training sample group;
computing a weighted value based on the sample rank, and weighting the diversity learning parameters by using the weighted value to obtain a target weighted value; and
computing a sum of the target weighted value and the difficulty learning parameters to obtain the update threshold.

**14.** The method according to claim 1, wherein after updating the training sample weight corresponding to each of the training samples based on the basic prediction model, the method further comprises:

acquiring current learning parameters, updating the current learning parameters according to a preset increment to obtain updated learning parameters, and treating the updated learning parameters as the current learning parameters.

**15.** A method for predicting data, comprising:

acquiring to-be-predicted data, the to-be-predicted data comprising to-be-predicted wild type protein information, to-be-predicted mutant type protein information and to-be-predicted compound information;

performing binding energy characteristic extraction based on the to-be-predicted wild type protein information and the to-be-predicted compound information to obtain to-be-predicted wild type energy characteristics, and performing binding energy characteristic extraction based on the to-be-predicted mutant type protein information and the to-be-predicted compound information to obtain to-be-predicted mutant type energy characteristics;

determining to-be-predicted target energy characteristics based on the to-be-predicted wild type energy characteristics and the to-be-predicted mutant type energy characteristics;

inputting the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight based on the basic prediction model, and returning to perform the operation of determining the current training sample from the training sample set based on the training sample weight; in a case of reaching the model training completion condition, the basic prediction model reaching the model training completion condition being treated as a target prediction model.

**16.** An apparatus for training a prediction model, comprising:

a sample acquisition module, configured to acquire a training sample set, the training sample set comprising a plurality of training samples, a training sample weight corresponding to each of the training samples and target energy characteristics corresponding to each of the training samples, each of the training samples comprising wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information;

a sample determination module, configured to determine a current training sample from the training sample set based on the training sample weight;

a training module, configured to input current target energy characteristics corresponding to the current training sample into a pre-trained prediction model for basic training, and obtaining a basic prediction model after completing the basic training; and

an iteration module, configured to judge whether a model training completion condition is reached or not; in a case of not reaching the model training completion condition, update the training sample weight corresponding to each of the training samples based on the basic prediction model, and return to perform the operation of determining the current training sample from the training sample set based on the training sample weight; in a case of reaching the model training completion condition, treat the basic prediction model reaching the model training completion condition as a target prediction model, the target prediction model being configured to predict interaction state information corresponding to the inputted protein information and the inputted compound information.

**17.** The apparatus according to claim 16, further comprising:

a pre-training module, configured to acquire each of the training samples and an interaction state tag corresponding to each of the training samples, each of the training samples comprising the wild type protein information, the mutant type protein information and the compound information; perform binding initial energy characteristic extraction based on the wild type protein information and the compound information to obtain wild type initial energy characteristics; perform binding initial energy characteristic extraction based on the mutant type protein information and the compound information to obtain mutant type initial energy characteristics, and determine target initial energy characteristics corresponding to each of the training samples based on the wild type initial energy characteristics and the mutant type initial energy characteristics; input the target initial energy characteristics corresponding to each of the training samples into an initial prediction model for prediction to obtain initial interaction state information corresponding to each of the training samples, the initial prediction model being established by using a random forest algorithm; perform loss computation based on the initial interaction state information corresponding to each of the training samples and the interaction state tag corresponding to each of the training samples to obtain initial loss information corresponding to each of the training samples; update the initial prediction model based on the initial loss information, and return to perform the operation of inputting the target energy characteristics corresponding to each of the training samples into the initial prediction model for prediction until completing the pre-training, thus obtaining characteristic importance corresponding to the pre-trained prediction model and the target initial energy characteristics; and determine the training sample weight corresponding to each of the training samples based on the loss information corresponding to each of the training samples in a case of completing pre-training, and select the target energy characteristics from the target initial energy characteristics based on the characteristic importance.

18. The apparatus according to claim 17, wherein the pre-training module is further configured to input the target initial energy characteristics corresponding to each of the training samples into the initial prediction model; and the initial prediction model is configured to treat the target initial energy characteristics corresponding to each of the training samples as a current to-be-divided set, and compute initial characteristic importance corresponding to the target initial energy characteristics; determine initial division characteristics from the target initial energy characteristics based on the initial characteristic importance; divide the target initial energy characteristics corresponding to each of the training samples based on the initial division characteristics so as to obtain each division result, the division result comprising target initial energy characteristics corresponding to each division sample; and treat each division result as the current to-be-divided set, and return to perform the operation of computing the initial characteristic importance corresponding to the target initial energy characteristics for iteration until completing division, thus obtaining initial interaction state information corresponding to each of the training samples.

19. The apparatus according to claim 16, wherein the sample acquisition module is further configured to acquire confidence corresponding to each of the training samples, and determine the training sample weight corresponding to each of the training samples based on the confidence.

20. The apparatus according to claim 16, wherein the sample acquisition module is further configured to perform binding energy characteristic extraction based on the wild type protein information and the compound information to obtain the wild type energy characteristics; perform binding energy characteristic extraction based on the mutant type protein information and the compound information to obtain the mutant type energy characteristics; and compute a difference between the wild type energy characteristics and the mutant type energy characteristics to obtain the target energy characteristics.

21. The apparatus according to claim 20, wherein the wild type energy characteristics comprise first wild type energy characteristics and second wild type energy characteristics; and the sample acquisition module is further configured to perform binding energy characteristic extraction by a non-physical scoring function based on wild type protein information and compound information to obtain the first wild type energy characteristics; perform binding energy characteristic extraction by a physical function based on the wild type protein information and the compound information to obtain the second wild type energy characteristics; and perform fusing based on the first wild type energy characteristics and the second wild type energy characteristics to obtain the wild type energy characteristics.

22. The apparatus according to claim 20, wherein the mutant type energy characteristics comprise first mutant type energy characteristics and second mutant type energy characteristics; and the sample acquisition module is further configured to perform binding energy characteristic extraction by a non-physical function based on the mutant type protein information and the compound information to obtain the first mutant type energy characteristics; perform binding energy characteristic extraction by a physical function based on the mutant type protein information and the compound information to obtain the second mutant type energy characteristics; and perform fusing based on the first mutant type energy characteristics and the second mutant type energy characteristics to obtain the mutant type energy characteristics.

23. The apparatus according to claim 16, wherein the sample determination module is further configured to acquire protein family information, and divide the training sample set based on the protein family information to obtain each training sample group; and select the current training sample from each training sample group based on the training sample weight to obtain a current training sample set; and

the training module is further configured to input the current target energy characteristics corresponding to each current training sample in the current training sample set into the pre-trained prediction model for basic training, and obtain a target basic prediction model after completing the basic training.

24. The apparatus according to claim 23, wherein the sample determination module is further configured to acquire current learning parameters, and determine a number of selected samples and sample distribution based on the current learning parameters; and select the current training sample from each training sample group according to the training sample weight based on the number of selected samples and the sample distribution to obtain a target current training sample set.

25. The apparatus according to claim 16, wherein the training module is further configured to input the current target energy characteristics corresponding to the current training sample into the pre-trained prediction model for prediction to obtain current interaction state information; compute an error between the current interaction state information and the interaction state tag corresponding to the current training sample to obtain current loss information; and update the pre-trained prediction model based on the current loss information, and return to perform the operation of inputting the current target energy characteristics corresponding to the current training sample into the pre-trained prediction model for prediction to obtain the current interaction state information so as to obtain the basic prediction model after reaching the basic training completion conditions.

26. The apparatus according to claim 16, wherein the iteration module is further configured to input the target energy characteristics corresponding to each of the training samples into the basic prediction model to obtain the basic interaction state information corresponding to each of the training samples; compute an error between the basic interaction state information corresponding to each of the training samples and the interaction state tag corresponding to each of the training samples to obtain basic loss information; and update the training sample weight based on the basic loss information to obtain an updated sample weight corresponding to each of the training samples.

27. The apparatus according to claim 26, wherein the iteration module is further configured to acquire current learning parameters, and compute an update threshold based on the current learning parameters; compare the update threshold with the basic loss information corresponding to each of the training samples to obtain a comparison result corresponding to each of the training samples; and determine the updated sample weight corresponding to each of the training samples according to the comparison result corresponding to each of the training samples.

28. The apparatus according to claim 27, wherein the current learning parameters comprise diversity learning parameters and difficulty learning parameters; and the iteration module is further configured to acquire each training sample group, determine a current training sample group from each training sample group, and compute a sample rank corresponding to the current training sample group; compute a weighted value based on the sample rank, and weight the diversity learning parameters by using the weighted value to obtain a target weighted value; and compute a sum of the target weighted value and the difficulty learning parameters to obtain the update threshold.

29. The apparatus according to claim 16, wherein the iteration module is further configured to acquire current learning parameters, update the current learning parameters according to a preset increment to obtain updated learning parameters, and treat the updated learning parameters as the current learning parameters.

30. An apparatus for predicting data, comprising:

a data acquisition module, configured to acquire to-be-predicted data, the to-be-predicted data comprising to-be-predicted wild type protein information, to-be-predicted mutant type protein information and to-be-predicted compound information;
a characteristic extraction module, configured to perform binding energy characteristic extraction based on the to-be-predicted wild type protein information and the to-be-predicted compound information to obtain to-be-predicted wild type energy characteristics, and perform binding energy characteristic extraction based on the to-be-predicted mutant type protein information and the to-be-predicted compound information to obtain to-be-predicted mutant type energy characteristics;
a target characteristic determination module, configured to determine to-be-predicted target energy characteristics based on the to-be-predicted wild type energy characteristics and the to-be-predicted mutant type energy characteristics; and
a prediction module, configured to input the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight based on the basic prediction model, and returning to perform the operation of determining the current training sample from the training sample set based on the training sample weight; in a case of reaching the model training completion condition, the basic prediction model reaching the model training completion condition being treated as a target prediction model.

31. A computer-readable storage medium, storing computer-readable instructions, the computer-readable instructions, when executed by a processor, implementing the operations of the method according to any one of claims 1 to 15.

32. A computer-readable storage medium, storing computer-readable instructions, the computer-readable instructions, when executed by a processor, implementing the operations of the method according to any one of claims 1 to 15.

33. A computer program product, comprising computer-readable instructions, the computer-readable instructions, when executed by a processor, implementing the operations of the method according to any one of claims 1 to 15.

FIG. 1

Step 202

Acquire a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information

Step 204

Determine a current training sample from the training sample set based on the training sample weight

Step 206

Input current target energy characteristics corresponding to the current training sample into a pre-trained prediction model for basic training, and obtain a basic prediction model after completing the basic training

Step 208

Complete model training

Yes

Step 208a

Obtain a target prediction model, the target prediction model being configured to predict interaction state information corresponding to the inputted protein information and the inputted compound information

No

Step 208b

Update the training sample weight corresponding to each of the training samples based on the basic prediction model

FIG. 2

Step 302

Acquire each of the training samples and an interaction state tag corresponding to each of the training samples, each of the training samples including the wild type protein information, the mutant type protein information and the compound information

Step 304

Perform binding initial energy characteristic extraction based on the wild type protein information and the compound information to obtain wild type initial energy characteristics

Step 306

Perform binding initial energy characteristic extraction based on the mutant type protein information and the compound information to obtain mutant type initial energy characteristics, and determine target initial energy characteristics corresponding to each of the training samples based on the wild type initial energy characteristics and the mutant type initial energy characteristics

Step 308

Input the target initial energy characteristics corresponding to each of the training samples into an initial prediction model for prediction to obtain the initial interaction state information corresponding to each of the training samples, the initial prediction model being established by using a random forest algorithm

Step 310

Perform loss computation based on the initial interaction state information corresponding to each of the training samples and the interaction state tag corresponding to each of the training samples to obtain initial loss information corresponding to each of the training samples

Step 312

Update the initial prediction model based on the initial loss information, and return to perform the operation of inputting the target energy characteristics corresponding to each of the training samples into the initial prediction model for prediction until completing the pre-training, thus obtaining characteristic importance corresponding to the pre-trained prediction model and the target initial energy characteristics

Step 314

Determine the training sample weight corresponding to each of the training samples based on the loss information corresponding to each of the training samples in a case of completing pre-training, and select the target energy characteristics from the target initial energy characteristics based on the characteristics importance

FIG. 3

Step 402

Input the target initial energy characteristics corresponding to each of the training samples into the initial prediction model

Step 404

Treat, by the initial prediction model, the target initial energy characteristics corresponding to each of the training samples as a current to-be-divided set, and compute initial characteristic importance corresponding to the target initial energy characteristics; determine initial division characteristics from the target initial energy characteristics based on the initial characteristics importance; divide the target initial energy characteristics corresponding to each of the training samples based on the initial division characteristics so as to obtain each division result, the division result including target initial energy characteristics corresponding to each division sample; and treat each division result as the current to-be-divided set, and return to perform the operation of computing the initial characteristics importance corresponding to the target initial energy characteristics for iteration until completing division, thus obtaining initial interaction state information corresponding to each of the training samples

FIG. 4

Step 502

Perform binding energy characteristic extraction based on the wild type protein information and the compound information to obtain wild type energy characteristics

Step 504

Perform binding energy characteristic extraction based on the mutant type protein information and the compound information to obtain mutant type energy characteristics

Step 506

Compute a difference between the wild type energy characteristics and the mutant type energy characteristics to obtain the target energy characteristics

FIG. 5

Step 602

Perform binding energy characteristic extraction by a non-physical scoring function based on the wild type protein information and the compound information to obtain the first wild type energy characteristics

Step 604

Perform binding energy characteristic extraction by a physical function based on the wild type protein information and the compound information to obtain the second wild type energy characteristics

Step 606

Perform using based on the first wild type energy characteristics and the second wild type energy characteristics to obtain the wild type energy characteristics

FIG. 6

Step 702

Perform binding energy characteristic extraction by a non-physical function based on the mutant type protein information and the compound information to obtain the first mutant type energy characteristics

Step 704

Perform binding energy characteristic extraction by a physical function based on the mutant type protein information and the compound information to obtain the second mutant type energy characteristics

Step 706

Perform fusing based on the first mutant type energy characteristics and the second mutant type energy characteristics to obtain the mutant type energy characteristics

FIG. 7

Step 802

Acquire protein family information, and divide the training sample set based on the protein family information to obtain each training sample group

Step 804

Select the current training sample from each training sample group based on the training sample weight to obtain a current training sample set

Step 806

Input the current target energy characteristics corresponding to each current training sample in the current training sample set into the pre-trained prediction model for basic training, and obtain a target basic prediction model after completing the basic training

FIG. 8

Step 902

Input the current target energy characteristics corresponding to the current training sample into the pre-trained prediction model for prediction to obtain current interaction state information

Step 904

Compute an error between the current interaction state information and the interaction state tag corresponding to the current training sample to obtain current loss information

Step 906

Update the pre-trained drug resistance prediction model based on the current loss information, and return to perform the operation of inputting the current target energy characteristics corresponding to the current training sample into the pre-trained prediction model for prediction to obtain the current interaction state information so as to obtain the basic prediction model after reaching basic training completion conditions

FIG. 9

Step 1002

Input the target energy characteristics corresponding to each of the training samples into the basic prediction model to obtain the basic interaction state information corresponding to each of the training samples

Step 1004

Compute an error between the basic interaction state information corresponding to each of the training samples and the interaction state tag corresponding to each of the training samples to obtain the basic loss information

Step 1006

Update the training sample weight based on the basic loss information to obtain an updated sample weight corresponding to each of the training samples

FIG. 10

_↗ Step 1102_

Acquire to-be-predicted data, the to-be-predicted data including to-be-predicted wild type protein information, to-be-predicted mutant type protein information and to-be-predicted compound information

_↗ Step 1104_

Perform binding energy characteristic extraction based on the to-be-predicted wild type protein information and the to-be-predicted compound information to obtain to-be-predicted wild type energy characteristics, and perform binding energy characteristic extraction based on the to-be-predicted mutant type protein information and the to-be-predicted compound information to obtain to-be-predicted mutant type energy characteristics

_↗ Step 1106_

Determine to-be-predicted target energy characteristics based on the to-be-predicted wild type energy characteristics and the to-be-predicted mutant type energy characteristics

_↗ Step 1108_

Input the to-be-predicted target energy characteristics into a target prediction model for prediction to obtain interaction state information; the target prediction model being obtained by following operations: acquiring a training sample set; determining a current training sample from the training sample set based on a training sample weight; inputting current target energy characteristics corresponding to the current training sample into a pre-trained prediction model for basic training, and obtaining a basic prediction model after completing the basic training; and judging whether a model training completion condition is reached or not; in a case of not reaching the model training completion condition, updating the training sample weight corresponding to each of the training samples based on the basic prediction model, and returning to perform the operation of determining the current training sample from the training sample set based on the training sample weight; in a case of reaching the model training completion condition, the basic prediction model reaching the model training completion condition being treated as a target prediction model

FIG. 11

Data | Protein
Wild type | Mutant type

Compound

Non-physical model characteristics

Physical and experience potential energy-based characteristics

Characteristic selection

Target prediction model

Predicate protein mutation which causes drug resistance

FIG. 12

Step 1302

Acquire a training sample set, the training sample set including a plurality of training samples, a training sample weight corresponding to each of the training samples and target energy characteristics corresponding to each of the training samples, each of the training samples including wild type protein information, mutant type protein information and compound information, the target energy characteristics being obtained based on wild type energy characteristics and mutant type energy characteristics, the wild type energy characteristics being obtained by performing binding energy characteristic extraction based on the wild type protein information and the compound information, and the mutant type energy characteristics being obtained by performing binding energy characteristic extraction based on the mutant type protein information and the compound information

Step 1304

Acquire protein family information, divide the training sample set based on the protein family information to obtain each training sample group, acquire current learning parameters, and determine a number of selected samples and sample distribution based on the current learning parameters, select the current training sample from each training sample group according to the training sample weight based on the number of selected samples and the sample distribution to obtain a target current training sample set

Step 1306

Input the target energy characteristics corresponding to each of the training samples in the target current training sample set into the basic prediction model to obtain the basic interaction state information corresponding to each of the training samples, and compute an error between the basic interaction state information corresponding to each of the training samples and the interaction state tag corresponding to each of the training samples to obtain the basic loss information

Step 1308

Compute a sample rank corresponding to each training sample group, compute a weighted value based on the sample rank, weight the diversity learning parameters by using the weighted value to obtain a target weighted value, and compute a sum of the target weighted value and the difficulty learning parameters to obtain the update threshold of each training sample group

Step 1310

Compare the update threshold with the basic loss information corresponding to the training sample in each training sample group to obtain a comparison result corresponding to each of the training samples, and determine the updated sample weight corresponding to the training sample in each training sample group according to the comparison result corresponding to the training sample

Step 1312

Update the current learning parameters according to the preset increment to obtain the updated learning parameters, treat the updated learning parameters as the current learning parameters, and return to perform the operation of determining the number of selected samples and the sample distribution based on the current learning parameters so as to obtain the target prediction module after completing the model training

FIG. 13

FIG. 14

FIG. 15

FIG. 16

FIG. 17

FIG. 18

FIG. 19

FIG. 20

## INTERNATIONAL SEARCH REPORT

| International application No. |
| --- |
| **PCT/CN2022/079885** |

**A. CLASSIFICATION OF SUBJECT MATTER**

G16B 40/00(2019.01)i

According to International Patent Classification (IPC) or to both national classification and IPC

**B. FIELDS SEARCHED**

Minimum documentation searched (classification system followed by classification symbols)

G16B

Documentation searched other than minimum documentation to the extent that such documents are included in the fields searched

Electronic data base consulted during the international search (name of data base and, where practicable, search terms used)

CNTXT, CNKI, ENTXT, DWPI: 模型, 训练, 预测, 蛋白质, 化合物, 突变, 目标, 样本, 权重, model, train, predict, protein, compound, mutant, target, sample, weight

**C. DOCUMENTS CONSIDERED TO BE RELEVANT**

| Category* | Citation of document, with indication, where appropriate, of the relevant passages | Relevant to claim No. |
| --- | --- | --- |
| PX | CN 112735535 A (TENCENT TECHNOLOGY SHENZHEN CO., LTD.) 30 April 2021 (2021-04-30) <br> entire document | 1-33 |
| A | CN 111667884 A (TIANJIN UNIVERSITY) 15 September 2020 (2020-09-15) <br> entire document | 1-33 |
| A | CN 110008984 A (ALIBABA GROUP HOLDING LIMITED) 12 July 2019 (2019-07-12) <br> entire document | 1-33 |
| A | CN 109147866 A (NANJING UNIVERSITY OF SCIENCE AND TECHNOLOGY) 04 January 2019 (2019-01-04) <br> entire document | 1-33 |
| A | CN 110443419 A (TAIYUAN UNIVERSITY OF TECHNOLOGY) 12 November 2019 (2019-11-12) <br> entire document | 1-33 |
| A | CN 111985274 A (SHENYANG INSTITUTE OF AUTOMATION, CHINESE ACADEMY OF SCIENCES) 24 November 2020 (2020-11-24) <br> entire document | 1-33 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **18 April 2022** | **09 May 2022** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.

**PCT/CN2022/079885**

**C.     DOCUMENTS CONSIDERED TO BE RELEVANT**

| Category* | Citation of document, with indication, where appropriate, of the relevant passages | Relevant to claim No. |
|---|---|---|
| A | US 2002019706 A1 (BRAUN, et al.) 14 February 2002 (2002-02-14)<br>entire document | 1-33 |

Form PCT/ISA/210 (second sheet) (January 2015)

**INTERNATIONAL SEARCH REPORT**
Information on patent family members

International application No.

**PCT/CN2022/079885**

| Patent document cited in search report | | | Publication date (day/month/year) | Patent family member(s) | | | Publication date (day/month/year) |
|---|---|---|---|---|---|---|---|
| CN | 112735535 | A | 30 April 2021 | CN | 112735535 | B | 25 June 2021 |
| CN | 111667884 | A | 15 September 2020 | None | | | |
| CN | 110008984 | A | 12 July 2019 | None | | | |
| CN | 109147866 | A | 04 January 2019 | None | | | |
| CN | 110443419 | A | 12 November 2019 | None | | | |
| CN | 111985274 | A | 24 November 2020 | None | | | |
| US | 2002019706 | A1 | 14 February 2002 | KR | 20010033817 | A | 25 April 2001 |
| | | | | EP | 1042669 | A1 | 11 October 2000 |
| | | | | JP | 2002500360 | A | 08 January 2002 |
| | | | | CA | 2316361 | A1 | 08 July 1999 |
| | | | | WO | 9934208 | A1 | 08 July 1999 |
| | | | | AU | 1950399 | A | 19 July 1999 |
| | | | | US | 6321164 | B1 | 20 November 2001 |
| | | | | EP | 1042669 | A4 | 08 December 2004 |
| | | | | US | 5708591 | A | 13 January 1998 |
| | | | | US | 6101449 | A | 08 August 2000 |
| | | | | US | 6564153 | B2 | 13 May 2003 |
| | | | | CA | 2316361 | C | 27 November 2007 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021103559296